# EUROPEAN PATENT SPECIFICATION

(11) **EP 2 321 343 B1**
(45) Date of publication and mention of the grant of the patent: **03.06.2015**
(21) Application number: 09762718.6
(22) Date of filing: 09.06.2009
(51) Int. Cl.: C07K 14/47

(54) **NOVEL RETINOID INDUCIBLE FACTOR AND USES THEREOF**
NEUER RETINOIDINDUZIERBARER FAKTOR UND ANWENDUNGEN DAVON
NOUVEAU FACTEUR INDUCTIBLE PAR LES RÉTINOÏDES ET APPLICATIONS ASSOCIÉES

(30) Priority: 09.06.2008 NO 20082550; 23.01.2009 NO 20090358
(43) Date of publication of application: 18.05.2011
(73) Proprietor: Bergen Teknologioverføring AS, 5006 Bergen (NO)
(72) Inventor: LILLEHAUG, Johan, R., 5052 Bergen (NO); PENDINO, Frederic, 92220 Bagneux (FR)
(74) Representative: Kröncke, Rolf
(86) International application number: PCT/NO2009/000214
(87) International publication number: WO 2009/151337

(56) References cited:
- DATABASE EMBL 28 February 2001 (2001-02-28), XP002557558 retrieved from EBI Database accession no. AX083504 & WO 01/12662 A (INCYTE GENOMICS INC [US]; LAL PREETI [US]; YUE HENRY [US]; TANG Y TOM) 22 February 2001 (2001-02-22)
- DATABASE EMBL 16 April 2001 (2001-04-16), XP002557559 retrieved from EBI Database accession no. BC006428
- DATABASE EMBL 9 March 2001 (2001-03-09), XP002557560 retrieved from EBI Database accession no. BC002490
- DATABASE UniProt 17 April 2007 (2007-04-17), XP002557561 retrieved from EBI Database accession no. Q7LFL8
- KATOH MASUKO ET AL: "Identification and characterization of human CXXC10 gene in silico" INTERNATIONAL JOURNAL OF ONCOLOGY, DEMETRIOS A. SPANDIDOS ED. & PUB, GR, vol. 25, no. 4, 1 October 2004 (2004-10-01), pages 1193-1199, XP008098319 ISSN: 1019-6439
- ROSENBAUER FRANK ET AL: "Transcription factors in myeloid development: balancing differentiation with transformation." NATURE REVIEWS. IMMUNOLOGY FEB 2007, vol. 7, no. 2, February 2007 (2007-02), pages 105-117, XP002557555 ISSN: 1474-1733
- AYTON PAUL M ET AL: "Binding to nonmethylated CpG DNA is essential for target recognition, transactivation, and myeloid transformation by an MLL oncoprotein." MOLECULAR AND CELLULAR BIOLOGY DEC 2004, vol. 24, no. 23, December 2004 (2004-12), pages 10470-10478, XP002557556 ISSN: 0270-7306
- PENDINO FRÉDÉRIC ET AL: "Functional involvement of RINF, retinoid-inducible nuclear factor (CXXC5), in normal and tumoral human myelopoiesis." BLOOD 2 APR 2009, vol. 113, no. 14, 2 April 2009 (2009-04-02), pages 3172-3181, XP002557557 ISSN: 1528-0020
- MAINGUY G ET AL: "A position-dependent organisation of retinoid response elements is conserved in the vertebrate Hox clusters" TRENDS IN GENETICS, ELSEVIER SCIENCE PUBLISHERS B.V. AMSTERDAM, NL LNKD- DOI:10.1016/S0168-9525(03)00202-6, vol. 19, no. 9, 1 September 2003 (2003-09-01), pages 476-479, XP004452640 ISSN: 0168-9525
- DATABASE EMBL [Online] 6 March 2002 (2002-03-06), XP002577351 retrieved from EBI Database accession no. AC113361
- MCGRANE ET AL: "Vitamin A regulation of gene expression: molecular mechanism of a prototype gene" JOURNAL OF NUTRITIONAL BIOCHEMISTRY, BUTTERWORTH PUBLISHERS, STONEHAM, GB LNKD- DOI:10.1016/J.JNUTBIO.2006.10.006, vol. 18, no. 8, 13 July 2007 (2007-07-13), pages 497-508, XP022153435 ISSN: 0955-2863
- SCORILAS ANDREAS: "Polyadenylate polymerase (PAP) and 3' end pre-mRNA processing: function, assays, and association with disease", CRITICAL REVIEWS IN CLINICAL LABORATORY SCIENCES, CRC PRESS, BACA RATON, FL, US, vol. 39, no. 3, 1 January 2002 (2002-01-01), pages 193-224, XP009134962, ISSN: 1040-8363
- ROCHEFORT H ET AL: "Cathepsin D: a protease involved in breast cancer metastasis", CANCER METASTASIS, KLUWER ACADEMIC PUBLISHERS, DORDRECHT, NL, vol. 9, no. 4, 1 December 1990 (1990-12-01), pages 321-331, XP009134964, ISSN: 0167-7659
- ASTORI AUDREY ET AL: "CXXC5 (retinoid-inducible nuclear factor, RINF) is a potential therapeutic target in high-risk human acute myeloid leukemia.", ONCOTARGET SEP 2013, vol. 4, no. 9, September 2013 (2013-09), pages 1438-1448, ISSN: 1949-2553
- GIAGOUNIDIS A A N ET AL: "Clinical, morphological, cytogenetic, and prognostic features of patients with myelodysplastic syndromes and del(5q) including band q31.", LEUKEMIA JAN 2004, vol. 18, no. 1, January 2004 (2004-01), pages 113-119, ISSN: 0887-6924

## Description

### FIELD OF THE INVENTION

The present invention relates to an in vitro method according to claim 1.

### BACKGROUND FOR THE INVENTION

Retinoids have anticancerous properties in many human tissues. These agents have particularly demonstrated their efficiency in the treatment of Acute Promyelocytic Leukemia (APL), a cancer disease that can be used as a model of responsiveness to these agents. Importantly, if retinoid receptors have well been indentified (RAR, RXR, PML-RAR) and extensively studied the last two decades, most of their target genes responsible for their antiproliferative and anticancer properties still remain to be identified. By a microarray approach, we have identified a new target gene of retinoids, CXXC5, encoding a nuclear factor that we have functionally characterized for the first time and named RINF (Retinoid-Inducible Nuclear Factor).

RINF expression seems to be required for terminal differentiation of leukemic cells triggered be retinoids. Indeed, RINF expression not only correlates with retinoid- induced differentiation of leukemic cells and with cytokine-induced myelopoiesis of normal CD34+ progenitors, but in addition, short hairpin RNA (shRNA) interference suggests for this gene a regulatory function in both normal and tumoral myelopoiesis. Also, RINF could play an important role in cancer. Interestingly, RINF gene localizes to 5q31.3, a small region often deleted in myeloid leukemia (acute myeloid leukemia [AML]/myelodysplasia [MDS]).

CXXC55 / RINF is known from Katoh Masuko et al., Internat. J. Oncol. vol. 25, 2004, pp. 1193-1199. This document mentions CXXC5 as being a paralog of CXXC4, and belonging to a family of proteins implicated in carcinogenesis and differentiation. However, the use of CXXC5 in the diagnosis of cancer is not disclosed.

Examples for the use of a diagnostic marker gene for cancer are known, as well. For example, Rochefort et al., Cancer Meatastasis, vol. 9, 1990, pp. 321-331 discloses cathepsin D as a diagnostic marker for breast cancer. However, again the use of CXXC5 in the diagnosis of cancer is not disclosed.

Differentiation of hematopoietic stem cells to terminally mature granulocytes is a multistage process requiring coordinate expression of genes orchestrated by lineage-restricted transcription factors. So far, a relatively small number of transcription factors have been demonstrated to be essential for hematopoiesis. Deregulation or mutations of these factors can switch the cell fate from differentiation to proliferation and contribute to Acute Myeloid Leukemia (AML), a group of malignant hemopathies characterized by a maturation arrest and an accumulation of immature blasts in the bone-marrow, blood, and other tissues.

The AML-M3 subtype (according to the French-American-British (FAB) classification) also known as Acute Promyelocytic Leukemia (APL), corresponds to clonal expansion of leukemic blasts blocked at the promyelocytic stage of granulocytic differentiation. This pathology whose genetic hallmark is the t(15;17) translocation, represents the first cancer treated by a transcription-based therapy reestablishing terminal differentiation. Indeed, in this pathology, pharmacological doses of all-*trans* retinoic acid (ATRA) trigger terminal maturation of leukemic blasts. At the molecular level, ATRA is known to act as a ligand for retinoic acid receptors (RARα, PML-RARα...) in APL cells and regulates transcriptional activation of downstream target genes. Despite extensive studies using an experimental model, the NB4 cell line, only a few target genes encoding transcription factors have so far been shown to be really essential for retinoid-induced-differentiation of promyelocytic cells.

### SUMMARY OF THE INVENTION

By using a microarray approach, we have identified several novel genes early induced by retinoids in NB4 cells and encoding transcription factors potentially involved in the re-establishment of terminal differentiation by ATRA. One of these genes induced by retinoid treatment encodes a nuclear factor that we named RINF (Retinoid-Inducible Nuclear Factor). RINF invalidation by RNA interference abrogates the differentiating action of ATRA in NB4 cells by enforcing a retinoid-resistance phenotype and delays cytokine-induced granulocytic differentiation of normal CD34+ myeloid progenitors, thus suggesting a key and general regulatory role for RINF in myeloid differentiation.

### DETAILED DESCRIPTION OF THE INVENTION

Herein retinoid-responsive nucleic acid are described, characterized in that it comprises the sequence of SEQ ID NO 1 and SEQ ID NO 2 or a functional fragment or variant thereof, or an functionally equivalent isolated DNA sequence hybridizable thereto, or a corresponding mRNA thereof.

Further a protein or protein derivative is described, characterized in that it comprises the sequence of SEQ ID NO 3 (CXXC5) or a functional fragment or variant thereof.

The use of said protein or a protein sequence, or said nucleic acid, for the manufacturing of a pharmaceutical composition for the prevention and/or treatment of various diseases is described.

Moreover the prevention and/or treatment of a hematopoietic disease, or improvement of differentiation of a hematopoietic cell in a mammal is described. A further embodiment relates to the impairment or blocking of differentiation and/or improvement of proliferation of a hematopoietic cell in vitro or in a mammal.

The hematopoietic cell can be a bone marrow cell, a peripheral blood cell, an umbilical cord blood cell, and the cell can be either tumoral or non-tumoral.

A preferred use is for re-establishment of differentiation in cells, such as lymphoid cells or acute myeloid leukemia cells.

The hematopoietic disease can be Myelodysplasia (MDS, myelodysplastic syndrome), Acute Myeloid Leukemia (AML), Acute Lymphoid Leukemia (ALL),

Myeloproliferative syndrome (MPS), Chronic Myeloid Leukemia (CML) or Chronic Lymphoid Leukemia (CLL).

Said cancer can be one of the cancer types selected from the group comprising leukemia, (Myelodysplasia (MDS, myelodysplastic syndrome), Acute Myeloid Leukemia (AML), Acute Lymphoid Leukemia (ALL), Myeloproliferative syndrome (MPS), Chronic Myeloid Leukemia (CML), Chronic Lymphoid Leukemia (CLL) and solid tumors (Breast cancer, melanoma, lung cancer, thyroid cancer, prostate cancer, neuroblastoma, and renal carcinoma).

A further aspect relates to the use of a retinoid to activate the expression of said nucleic acid, and/or to enhance the expression of said protein or protein sequence in a mammal in need thereof. A preferred embodiment is ATRA.

A further aspect relates to a method of regulating the expression of CXXC5 by retroviral or lentiviral vectors (over-expression) or by shRNA molecules (repression).

The invention relates to a method for diagnosis of a cancer disease or a hematopoietic disease as defined in claim 1.An embodiment relates to the diagnosis of these diseases, using an antibody for the diagnosis.

### DEFINITIONS

### Hematopoiesis:

Hematopoiesis (from Ancient Greek: haima blood; poiesis to make), sometimes also called hemopoiesis, is the formation of blood cellular components (erythrocytes, thrombocytes, granulocytes (neutrophiles, basophils, eosinophiles), monocytes, macrophage, and lymphocytes (B, T and NK). All cellular blood components are derived from hematopoietic stem cells.

### Hematopoietic cell:

Any cell from the hematopoietic tissue (including lymphoid, myeloid cell). This cell can be a stem cell (HSC), a progenitor cell (common progenitor for myeloid or lymphoid), a committed cells or a terminally differentiated blood cell.

Myelopoiesis: Formation of myeloid cells from the pluripotent hematopoietic stem cells in the bone marrow via myeloid stem cells. Myelopoiesis generally refers to the production of leukocytes in blood, such as monocytes and granulocytes. This process also produces precursor cells for macrophage and dendritic cells found in the lymphoid tissue. In hematology, the term "myeloid cell" is used to describe any leukocyte that is not a lymphocyte and then also include erythrocytes (red blood cells) and thrombocytes (platellet) in addition to granulocytes, monocytes, macrophages and dendritic cells.

### Acute Myeloid Leukemia (AML):

Acute myeloid leukemia (AML), also known as acute myelogenous leukemia, is a cancer of the myeloid line of white blood cells, characterized by the rapid proliferation of abnormal cells which accumulate in the bone marrow and interfere with the production of normal blood cells. The symptoms of AML are caused by replacement of normal bone marrow with leukemic cells, resulting in a drop in red blood cells, platelets, and normal white blood cells. These symptoms include fatigue, shortness of breath, easy bruising and bleeding, and increased risk of infection. AML is characterized by a maturation clock. According to the French-American-British (FAB) classification, 8 subtypes of AML can be distinguished (from M0 to M7) based on the stage at which the differentiation is blocked, the hematopoietic compartment concerned, and the degree of maturity of the leukemic cells.

The "status" of a gene or protein means the sequence analysis or the expression level of the gene or protein, and the number of copies of a gene, and methylation status.

Retinoids: A class of chemical compounds that are related structurally or functionally to vitamin A. In the present application, the term retinoid means any compound able to bind to and activate retinoic acid receptors. These receptors bind Retinoic Acid-Responsive Elements (RARE) present in the promotors of their direct target genes and usually activate their transcription after binding with their ligand (for instance retinoic acid).

Here, a retinoid-responsive gene or protein, is a gene or a protein whose level of expression is induced upon treatment with a retinoid (like retinoic acid).

### Prognosis for retinoid responsiveness:

Only a small percentage of leukemic or cancer cells respond to therapy with retinoids. Moreover, the clinical response to this therapy usually requires days or weeks of treatment before having any beneficial effect for the patient. The existence of an early gene or protein biomarker of retinoid responsiveness, that could predict the latter outcome of the treatment of the disease with these agents, would consitute an important prognosis indicator that would help clinicians in deciding if their patients should undertake such a treatment or another one.

### EXPERIMENTAL SECTION

### Materials and methods

### Cell culture, treatments, and RNA preparation.

Human breast carcinoma cells (MCF7) and myeloid cells (NB4, NB4-LR1, NB4-LR2, K562, LAMA-84 and HL60) were cultured in RPMI 1640 medium (Invitrogen) supplemented with 10% foetal bovine serum (Biochrom AG), 2 mM L-Glutamine, 50 units/ml penicillin G and 50 µg/ml streptomycin (Invitrogen) and were incubated at 37°C in the dark, in a 5% CO₂/humidified atmosphere. For *in vitro* expansion of human bone marrow primary CD34+ cells (StemCell technologies), we supplemented the above medium with 20 ng/mL of Interleukin 3 (IL3), 20 ng/mL of Granulocyte-colony stimulating factor (G-CSF) and 50 ng/mL of Stem cell factor (SCF) purchased from Peprotech. Maturation was evaluated by morphology with May-Grünwald-Giemsa (MGG) staining and by nitroblue tetrazolium (NBT) reduction assay as previously described in Duprez E, Ruchaud S, Houge G, et al. A retinoid acid 'resistant' t(15;17) acute promyelocytic leukemia cell line: isolation, morphological, immunological, and molecular features. Leukemia. 1992;6:1281-1287. Cell density was determined using a Coulter Counter (Beckman). Cell proliferation was represented as Population Doublings (PD) calculated by the formula: *PD = Log (N*/*No)*/*Log2,* where N is the number of cells counted and *No* the number of cells seeded at day 0. Cells treated or not with ATRA (Sigma) were collected together and directly stored at -80°C for RNA preparation with Trizol (Invitrogen) or RNeasy mini kit (Qiagen). Yield and quality of the extracted RNA was evaluated by NanoDrop® ND-1000 spectrophotometer (NanoDrop Technologies).

### Microarray hybridization

All microarray experiments were performed using the Applied Biosystems (AB) Expression Array system, which is based upon chemiluminescence detection. The AB human microarray contains 31,700 oligonucleotide probes (60-mers) representing 27,868 individual human genes. Before labelling, amount and quality of the extracted RNA was verified by NanoDrop® ND-1000 spectrophotometer and Agilent 2100 Bioanalyzer (Agilent technologies). Two µg of total RNA from each sample were converted into digoxigenin (DIG)-labelled cRNA (with DIG-dUTP) using the AB Chemiluminescent RT-IVT labelling kit version 2.0 (PN 4363252, Roche). Amount (50-70 µg) and quality of the DIG-labelled cRNA was controlled by NanoDrop spectrophotometer and Agilent 2100 Bioanalyzer. Twenty µg of DIG-labelled cRNA was hybridized to the AB Human Genome Survey Microarray version 1.0 according to the manufacturer's instructions. The chemiluminescent signal detection, image acquisition and image analysis of the microarrays were performed on the AB 1700 Chemiluminescent Microarray Analyser (PN 4338036) following the manufacturer's protocol (PN 4339629). Images were auto-gridded and the chemiluminescent signals were quantified, corrected for background, and finally, spot-and spatially-normalized using the AB 1700 Chemiluminescent Microarray Analyzer software v1.03 (PN 4336391). A total of 6 microarrays were used for the analysis. Two replicates (independent labelling and independent hybridization process) were generated for NB4 samples at 3 hours. For inter-array normalization, we applied global median normalization across all microarrays to achieve the same median signal intensities for each array. MIAME compliant documentation of the microarray experiments have been deposited in Array Express at the European Bioinformatics Institute (www.ebi.ac.uk/arrayexpress) under the accession number E-BASE-7.

### Microarray data analysis and gene classification

The Applied Biosystems Expression System software was used to extract signals and signal-to-noise ratios (S/N). Only microarrays showing an average normalised signal intensity above 5,000 and a median background below 600 were included in the study. Signal intensities were imported into J-Express Pro V2.7 software (MolMine, Bergen, Norway) in accordance with Dysvik B, Jonassen I. J-Express: exploring gene expression data using Java. Bioinformatics. 2001;17:369-370., where inter-array quantile normalisation was performed in order to minimise the effect of external variables introduced into the data. When relevant, quality filtering of unreliable spots (S/N<3) was performed before normalisation. All the genes identified were classified using PANTHER™ (Protein ANalysis THrough Evolutionary Relationships) and Gene Ontology™ (GO).

### Quantitative RT-PCR

First-strand cDNA synthesis (RT) were carried out starting with total RNA (0,1 to 1 µg) in a 20-µl volume using oligo-dT primers with Transcriptor Reverse Transcriptase (Roche) in accordance with the manufacturer's instructions. Quantitative PCRs were performed using SYBRgreen detection kit on a Light Cycler 480 machine (Roche) in accordance with the manufacturer's instructions. For each gene (cxxc5, cd34, gcsfr and cd11b), relative mRNA expressions were normalized to rpP2 gene expression. Primers for detection of *cxxc5* (5'*-tccgctgctctggagaag*-3' and 5'-*cacacgagcagtgacattgc-3'), rpP2* (5'-atgcgctacgtcgcc-3' and 5'-ttaatcaaaaaggccaaatcccat-3'), cd34 (5'-*cagctggagccccacag*-3' and 5'-*gaggtcccaggtcctgagc-3'),* gcsfr *(5'-gtgcccacaatcatggaggag-3'* and 5'-*catcctcctccagcactgtg*-3'), and cd11 b (5'*-ctgctcctggccctcatc*-3' and 5'-*gacccccttcactcatcatgtc*-3') were all designed to be used in the same conditions of real-time PCR amplification: denaturation at 95°C, 10 seconds; annealing at 58°C, 10 seconds; elongation at 72°C, 12 seconds.

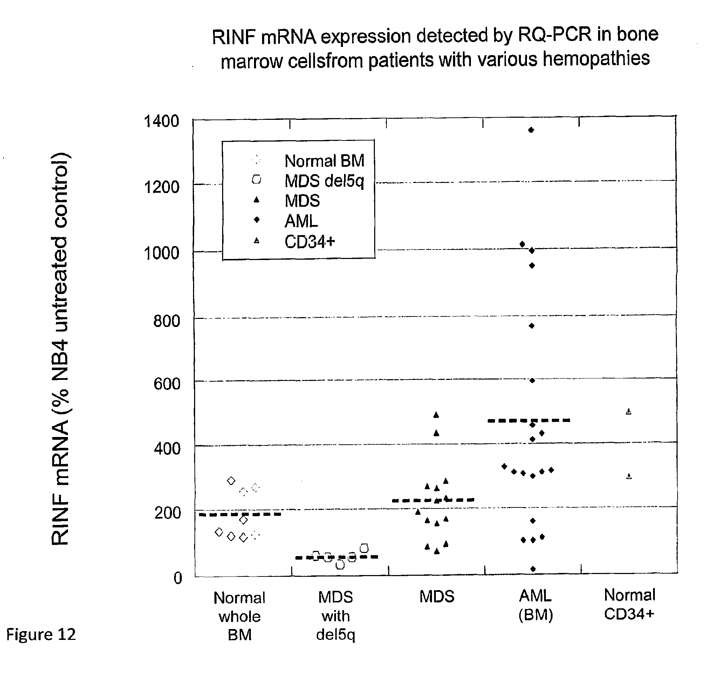

### For the results given in the figures 12,13 and 14, the quantitative RT-PCR uses Patients Materials.

A series of 105 bone marrow or blood samples were collected from 94 patients suffering from various hemopathies and 13 healthy donors. The various pathologies were classified according to the French-American-British (FAB. Bennet et al. 1982) and WHO (Vardiman et al. 2002) classifications as followed: 5 MDS with chromosome 5q deletion, 14 MDS without chromosome 5q deletion, 20 AML (bone marrow samples) and 37 AML (blood samples), 14 ALL B (blood) and 2 ALL T (blood). All the patients signed an informed consent.

### Quantitative RT-PCR of RINF in patient samples (figures 12, 13 and 14)

First-strand cDNA synthesis (RT) were carried out starting with total RNA (0,1 to 1 µg) in a 20-µl volume using oligo-dT primers and random hexamer primers with Transcriptor Reverse Transcriptase (Roche - 05 531 287 001) in accordance with the manufacturer's instructions. Quantitative PCRs were performed using specific Hybridization probes targeting CXXC5 gene on a Light Cycler 480 machine (Roche) in accordance with the manufacturer's instructions of the kit Lightcycler® 480 ProbesMaster (04 707 494 001). Relative mRNA expressions were normalized to rpP2 gene expression. Primers for detection of *cxxc5* (5'*-tccgctgctctggagaag*-3', 5'-*cacacgagcagtgacattgc*-3' and 6FAM-AACCCAAAgCTgCCCTCTCC-BBQ), *rpP2* (5'-atgcgctacgtcgcc-3', 5'-ttaatcaaaaaggccaaatcccat-3' and Cy5-AgCTgAATggAAAAAACATTgAAgACgTC-BBQ), were all designed to be used in the same conditions of real-time PCR amplification after a initial denaturation at 95°C during 5 min, and then proceed during 44 cycles as followed: denaturation for 10 seconds at 95°C; and elongation at 55°C for 20 seconds.

### Sequencing CXXC5

Prior to sequencing PCR products were subjected to purification using ExoSAP - IT kit (GE healthcare - 78201) according to manufactures instructions. Sequencing was done using BigDye® v3.1 cycle sequencing Kit (ABI - 4337456) with specific forward (5'- gcacaaaagtggtgctgtg- 3') and reverse - (5'- gcgtggtgcaggagcat- 3') sequencing primers in a total volume of 10µl with the following reaction conditions: denatuation 96°C, 10 seconds ; annealing at 50°C, 5 seconds; elongation at 60°C, 4 minutes and run for 25 cycles.

### Solid tumors

Concerning solid tumors, we investigated the RINF mRNA expression level in 35 samples from patients suffering from breast cancer (and 7 healthy controls), 40 samples from patients suffering from metastatic melanoma (and 8 healthy nevi controls) and 28 thyroid cancer samples containing both tumor and benign match pair.

### Chromatin Immuno-Precipitation experiments

Twenty millions of NB4 cells were crosslinked with formaldehyde (1% v/v) in RPMI medium (Invitrogen) for 10 minutes at 37°C, rinsed twice with ice-cold PBS, resuspended in hypotonic cell lysis buffer (0.25% Triton X-100, 10 mM Na-EDTA, 0.5 mM Na-EGTA, 10 mM Tris-HCl, pH 8.0, and protease inhibitor cocktail). Plasma membranes were broken using a Dounce (20 strokes) and collected nuclei (5 minutes centrifugation at 650g, 4°C) were resuspended in 1200µl of ChIP buffer (0.1% SDS, 0.1% Na-Deoxycholate, 1% Triton x-100, 1mM EDTA, 140mM NaCl, 10mM Tris pH 8, and protease inhibitor cocktail), and then sonicated to obtain DNA fragments of 500-1000 bp in length. For each IP, 200 µl of the sonication product was harvested and diluted 10 folds in the dilution buffer (20mM Tris pH 8, 2mM EDTA, 150mM NaCl, 1% Triton x-100). To reduce non specific binding, a preclearing step was performed before hybridization by adding 70µl of salmon sperm DNA/Protein A Agarose 50% slurry (Upstate) for 2h at 4°C on a rotating plate. Hybridization was performed (overnight at 4°C) by adding 4µg of an anti-RARα antibody (Abcam-H1920), or anti-PML antibody (Santa Cruz, sc-966) to the fragmented chromatin mixture for immunoprecipitation (IP). Immunoprecipitation was performed by adding 70µl of salmon sperm DNA/Protein A Agarose 50% slurry (Upstate) for 4h at 4°C on a rotating plate. The immunocomplex was recovered by centrifugation and eluted in 200 µl of elution buffer (1% SDS, 100mM NaHCO3) after extensive washing. To remove DNA-protein crosslinks, 8µl of 5M NaCl were added to the eluates, followed by heating at 65°C overnight. The fractions were then treated with RNAse A (10 µg/ml) and proteinase K at 42°C for 2h. The resultant DNA from each IP was purified by phenol/chloroform extraction and resuspended in 40 µl of TE (10mM Tris-HCl, pH 8.0, 1 mM EDTA, pH 8.0. For the input fraction, 20 µl of the crosslinked chromatin was saved after the preclearing step, diluted 10 folds in H2O, and was subjected to the same steps for removing DNA-proteins crosslinks. Input, IP and No Ab fractions were analysed by PCR using pairs of primers that encompass the Retinoic Acid Responsive Elements located in promoter regions of *RINF* or *RARb2.* Primers for *RINF* promoter (105 bp) 5'*-gcagtctgagatggttcccagg-*3'/5'*-tgcatgtaccattccctctgcc*-3' and for *RARb2* promoter (247 bp) 5'-*tcctgggagttggtgatgtcag-*3'/5'-*aaaccctgctcggatcgctc* -3'.

### Preparation of cytoplasmic and nuclear fractions.

Cell fractionation was performed at 4°C. NB4 cells were collected by centrifugation at 200g, washed twice with PBS, suspended in the hypotonic lysis buffer containing proteases and phosphatases inhibitors (25 mM Tris-HCl pH 7.5, 12.5 mM NaF, 0.2 mM sodium orthovanadate, 1 % protease inhibitor cocktail, Sigma P8340) and allowed to swell during 40 minutes. The plasma membranes were broken by homogenisation of the cell suspension with a conic pestle in a microfuge tube (Eppendorf). Triton-X 100 was added at 0.1 % final concentration just before centrifugation at 1,000g during 3 minutes. The supernatant consisting of the cytoplasmic fraction, is separated from the pellet consisting of the nuclei. The nuclei were washed twice with the lysis buffer containing proteases and phosphatases inhibitors, recovered by centrifugation at 1,000g and extracted with 4x sample buffer (Laëmmli 1970) for 8 minutes at 100 °C.

### Western-blot.

Western blotting was carried out as previously described by Wu YL, Dudognon C, Nguyen E, et al. Immunodetection of human telomerase reverse-transcriptase (hTERT) re-appraised: nucleolin and telomerase cross paths. J Cell Sci. 2006;119:2797-2806.

Customized rabbit polyclonal peptide-specific antibody against RINF was produced by Biogenes GmBH. The immunogen peptide corresponds to amino acids 45-58 of RINF protein. Antibody specificity was confirmed by competitive inhibition of the western-blot signal by addition of the immunogene peptide to the primary antibody solution. Briefly, blots were incubated with primary antibody against RINF (polyclonal antibody), PARP (monoclonal mouse IgG, Calbiochem, n°AM30) or actin (polyclonal rabbit IgG, Sigma, n°A2066) and then with an appropriate peroxydase conjugated secondary antibody. Detection of proteins was performed using a chemiluminescent detection system (Amersham Pharmacia Biotech). Blot with human tissue extracts was purchased from Millipore (TB300).

### Preparation of cytoplasmic and nuclear fractions.

Cell fractionation was performed at 4°C. NB4 cells were collected by centrifugation at 200g, washed twice with PBS, suspended in the hypotonic lysis buffer containing proteases and phosphatases inhibitors (25 mM Tris-HCl pH 7.5, 12.5 mM NaF, 0.2 mM sodium orthovanadate, 1 % protease inhibitor cocktail, Sigma P8340) and allowed to swell during 40 minutes. The plasma membranes were broken by homogenisation of the cell suspension with a conic pestle in a microfuge tube (Eppendorf). Triton-X 100 was added at 0.1 % final concentration just before centrifugation at 1,000g during 3 minutes. The supernatant consisting of the cytoplasmic fraction, is separated from the pellet consisting of the nuclei. The nuclei were washed twice with the lysis buffer containing proteases and phosphatases inhibitors, recovered by centrifugation at 1,000g and extracted with 4x sample buffer (Laëmmli 1970) for 8 minutes at 100 °C.

### Immunofluorescence.

Plasmid encoding FLAG-tagged RINF was constructed from NB4 cells cDNA. PCR products were inserted into the pFLAG-CMV-4 expression vector (Sigma). MCF7 cells were grown on coverslips and transfected with FLAG-tagged-RINF constructs according to Fugene HD manufacturer's protocol (Roche). Two days post-transfection, cells were washed once in PBS and fixed for 15 minutes in 2% paraformaldehyde. Fixed cells were washed three times in PBS, permeabilized in 0.1% Triton X-100 for 10 minutes and then incubated in blocking buffer (PBS, 2% BSA) for 30 minutes. Cells were then incubated overnight with the primary antibody (rabbit polyclonal anti-Flag M2, Sigma) diluted 1:3,000 in blocking buffer (PBS, 2% BSA), washed twice in PBS and incubated with the secondary antibody (Alexa-488-conjugated anti-rabbit from Molecular Probes, Invitrogen) diluted 1:1,000 (PBS, 2% BSA), for 1 hour at room temperature. Subsequently, cells were washed three times in PBS and were mounted in Vectashield mounting medium with 4',6-diaminidine-2-phenylindole (DAPI, Vector Laboratories) to counterstain nuclei. Immunofluorescent images were acquired by confocal microscopy on a Zeiss LSM510 META confocal laser microscope with a Plan Apochromat 63X N.A.1.4 oil-immersion objective using the LSM510 software v4.0 (Zeiss).

### Plasmids for lentiviral infections.

Lentiviral plasmids (pLKO.1/shRNA/RINF) targeting *RINF* expression were purchased from Sigma (MISSION® shRNA Bacterial Glycerol Stock) and control vectors (pLKO.1/TRC and pLKO.1/shRNA/scramble controls) were kindly provided by David Root and David M. Sabatini through a material transfer agreement (Addgene plasmids 10879 and 1864). Briefly, production of lentiviral particles were performed by transient co-transfection (Fugene HD, Roche) of HEK 293T cells with the 2nd generation packaging system (e.g. packaging plasmid psPAX2 and envelope plasmid pMD2.G) developed by Trono's lab (Addgene plasmids 12260 and 12259). Viral supernatants were harvested and filtered two days post-transfection and then applied to growing cells for spin-infection (2400 rpm, 1 h at room-temperature), which was carried out in presence of proteamine sulfate (5[mu]g/ml_). Two days post-infection, NB4 cells were selected for at least 2 days with puromycine (Sigma) at 1 [mu]g/mL.

### Plasmids and Retroviral Infections

The murine stem cell virus retroviral vector Mig-R1 , containing encephalomyocarditis virus internal ribosomal entry sequence and green fluorescent protein (GFP) as a reporter gene, was gently provided by W. S. Pear (University of Pennsylvania, Philadelphia, PA). RINF was inserted into Mig-R1 so that the 5' viral long terminal repeat (LTR) promoter drives its expression. The Mig-R1 constructs (Mig-R1/empty and Mig-R1/RINF) were transfected into the Phoenix retroviral packaging cell line to produce (VSV-G pseudotyped) viral supernatants that were harvest 2 days post- transfections. Infections, were then carried out in the presence of 4 [mu]g/ml of proteamine sulfate. Infected cells were sorted nine days after infection for GFP fluorescence.

### URLs

Exon-intron structure and genomic organization of Cxxc[delta] gene was performed using fast DB (www.fast-db.com) in accordance with de la Grange P, Dutertre M, Martin N, Auboeuf D. FAST DB: a website resource for the study of the expression regulation of human gene products. Nucleic Acids Res. 2005;33:4276-4284. Theoretical molecular mass of RINF protein was calculated at ExPASy (Expert Protein Analysis System) proteomic server website (www.expasy.ch/tools/pi_tool.html). In silico analysis of the putative NLS motif was performed using PredictNLS (cubic.bioc.columbia.edu/predictNLS/).

### Results

The results of the experiments will now be further described, with reference to the following figures:

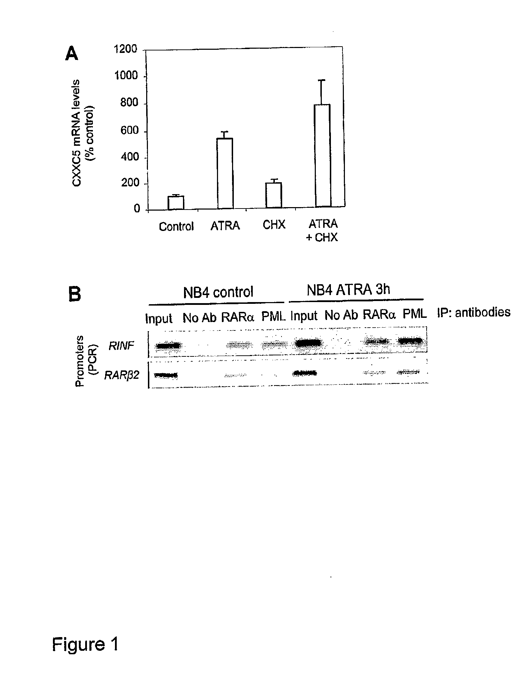

Figure 1 shows that *CXXC5* (*RINF*) is a direct target of retinoic acid. (A). RINF mRNA expression levels were measured by quantitative RT-PCR after 4h of ATRA-treatment (1 µM). Inhibition of translation with cycloheximide (CHX) used at 10 µg/mL did not block ATRA-induced increase in RINF mRNA level, demonstrating that this process does not require *de novo* protein synthesis, and strongly suggests that *Rinf* is a primary target of retinoic acid. The histogram represents means from two independent cell culture treatments (+/- s.e.m). (B). *In vivo* binding of RARα and PML-RARα to the *RINF* promoter in NB4 cells. Crosslinked chromatin was prepared from NB4 cells treated or not with 1µM ATRA for 3h and immunoprecipitated with anti- RARα or anti-PML antibodies. The precipitates were subjected to PCR analysis using primer pairs spanning the human *RINF* or *RARb2* gene promoters. The primers were designed to encompass the putative retinoid responsive element found in the *RINF* promoter (*gg**agttca**tg**aggtga**gc*) or the well established RARE (***ggttca**ccgaa**agttca***) in *RARb2* promoter (here used as a positive control). Input: PCRs performed on total chromatin from NB4 cells before IP. No Ab (No Antibody control): PCRs performed on sample obtained after IP with an irrelevant antibody or without any antibody.

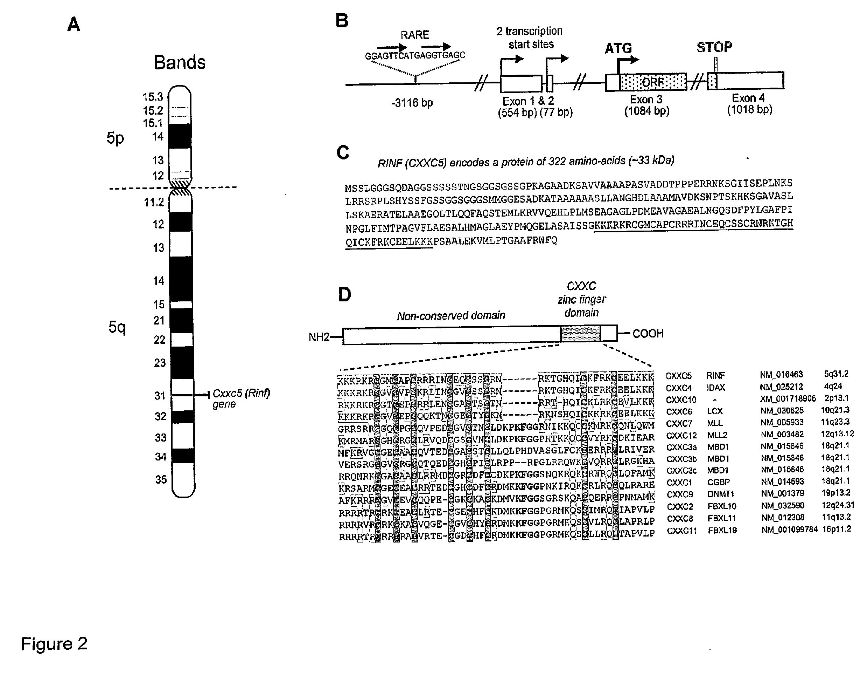

Figure 2 shows the *Cxxc5* gene structure and protein product (RINF). (A) The *Cxxc5* (*Rinf*) gene localizes to chromosome 5q31.3. The gene starts 139,008,130 bps from *pter* and ends 139,043,651 bps from *pter.* It is orientated in *plus* strand direction and is 35,522 kbps long. (B) Genomic organization of *Cxxc5* (*Rinf*) gene. The gene is organized in 4 exons and 95% of the open reading frame is located in exon 3. A putative Retinoic Acid Responsive Element (RARE) of Direct Repeat 2 (DR2) type is situated at -3116 bps up-stream of the exon 1 transcription start site. (C) Predicted amino acid sequence of RINF according to one letter amino acid representation. The open reading frame predicts a protein sequence of 322 amino acid residues and a theoretical molecular weight of 32.98 kDa (www.expasy.ch/tools/pi_tool.html). The zinc finger domain is underlined. (D) Except the CXXC zinc finger domain, no conserved structural domain was found. An alignment of the CXXC-motif of RINF protein with its human paralogs is presented. Amino acids that are invariant among CXXC-motifs are in grey and the conserved cysteine residues are in red. The consensus sequence for CXXC-type zinc finger can be defined by *Cx2Cx2Cx4-5Cx2Cx2C9-15Cx2Cx4C.*

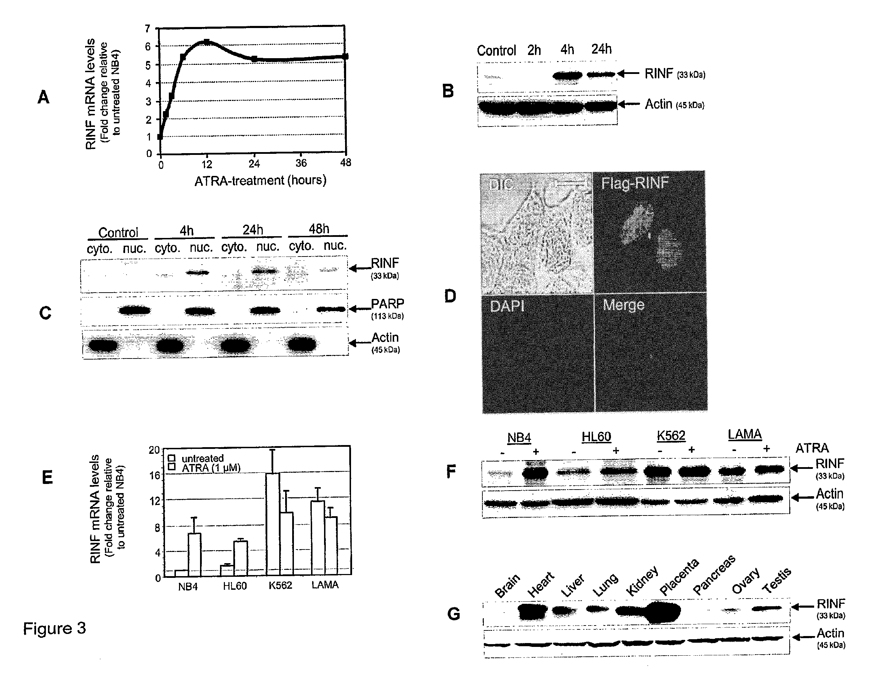

Figure 3 shows the RINF expression and subcellular localization in NB4 cells and other myeloid cell lines and tissues. (A) Relative expression of RINF mRNA levels (measured by quantitative RT-PCR) during ATRA treatment (1 µM) of NB4 cells. (B) Expression of RINF protein in total extracts from NB4 cells treated or not with ATRA (1 µM). RINF was detected with our customized polyclonal rabbit antibody (see Methods) that detects a specific band at 33 kDa. Actin was used as a loading control. (C) Expression of RINF protein in nuclear and cytosolic fractions of NB4 cells treated or not with ATRA (1 µM). RINF was detected with the polyclonal antibody. In this experiment, PARP and Actin were used as controls to evaluate quality, purity, and loading of the nuclear and cytosolic extracts. (D) FLAG-tagged RINF (green; Alexa Fluor 488-conjugated anti-FLAG monoclonal antibody) localizes in the nucleus (stained in blue with DAPI) of MCF7 cells analyzed by confocal and Differential Interference Contrast (DIC) microscopy. Scale bar, 10 µm. (E) RINF mRNA expression in various myeloid cell lines measured by quantitative RT-PCR with or without ATRA at 4h of treatment (see Methods). Expression of RINF protein in various myeloid cell lines (F) treated or not with ATRA (1 µM, during 4h), and in various human tissues (G). The same amounts of protein (determined with BCA assay test) were loaded for each of the myeloid cell line (20 µg) and human tissue extracts (65 µg, see Methods). Actin was used as a loading control.

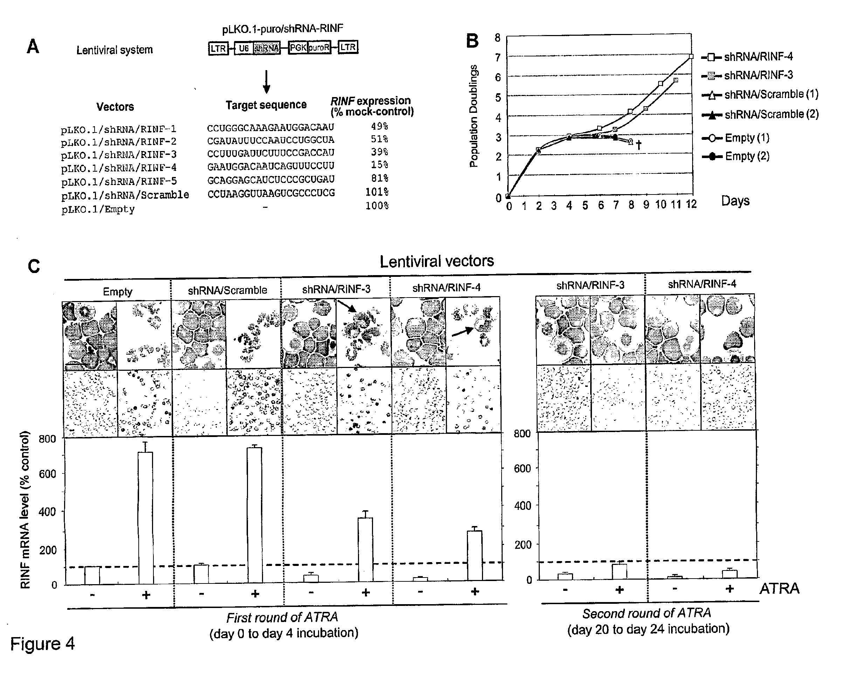

Figure 4 shows that shRNA-mediated silencing of *Rinf* imparts resistance to ATRA-induced terminal differentiation of NB4 cells. (A) Lentiviral shRNA vectors and their mRNA target sequences used to knock down *Rinf* expression. After infection and selection of NB4 cells (see Methods) with the lentiviral vector constructs, their efficiencies to target basal Rinf expression were monitored by quantitative RT-PCR measuring basal *RINF* mRNA expression levels (indicated in % of mock control, the pLKO.1/Empty vector). The most efficient knockdowns were obtained with shRNA/RINF-3 and shRNA/RINF-4 constructs (61% and 85% respectively, in the absence of ATRA). (B) Cell growth (population doublings) of NB4 cells, stably expressing the shRNA-constructs, in the presence of ATRA (1µM). The presented kinetic experiment (here during 12 days of ATRA-treatment) is representative of three separate experiments (performed from different batches of infections). Post-maturation cell death of control cells is indicated by a †. (C) *RINF* mRNA expression assessed by quantitative RT-PCR (% of untreated mock-control at 12 hours of culture) and terminal differentiation assessed by cell morphology at day 4 (scale bar, 25 µm) and NBT reduction assay assessed at day 2 (scale bar, 25 µm) of NB4 cells infected with Empty, hRNA/Scramble, shRNA/RINF-3 and shRNA/RINF-4 vectors. Cells were treated or not with ATRA (1µM) for four days (first round of ATRA, d0-4, left panel). After 2 more weeks of culture in the absence of ATRA, shRNA/RINF-3 and shRNA/RINF-4 cells that escaped the first round of ATRA were retreated (second round of ATRA, day 20 to 24, right panel) for four days with ATRA (1µM).

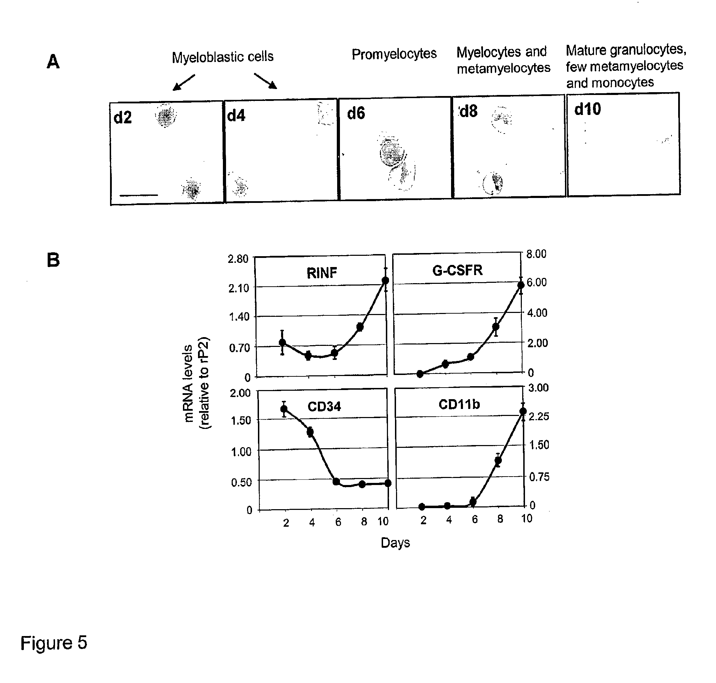

Figure 5 shows the RINF expression during normal myelopoiesis. (A) Cytokine-induced (IL3 and G-CSF at 20 ng/mL, SCF at 50 ng/mL) granulocytic differentiation of myeloid CD34+ cells (from a healthy donor). For cell morphology (scale bar, 25 µm), cells were spread on a glass slide by cytospin, air-dried, and stained with May-Grünwald Giemsa (MGG) at different time points of culture (from day 2 to day 10). For each day of culture recorded, the main stages of myelopoïesis observed is indicated. At day 10, most of the cells were terminally differentiated into polynuclear neutrophil granulocytes. In our experimental conditions, a few monocytic cells were also noticed (not, shown). (B) Relative mRNA expression of various genes (measured by quantitative RT-PCR) during cytokine-induced granulocytic differentiation of CD34+ progenitors (+/- s.e.m).

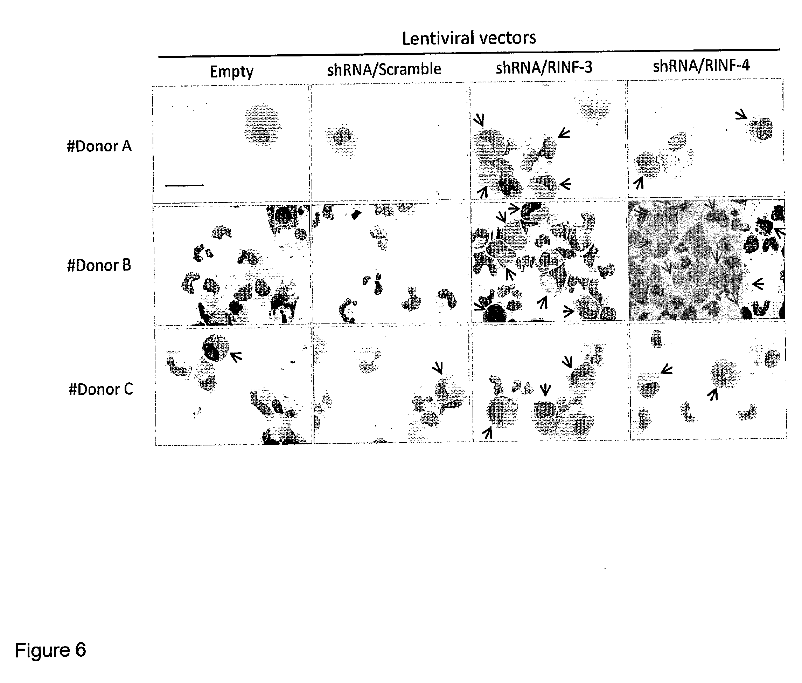

Figure 6 shows the functional involvement of RINF during normal myelopoiesis. CD34+ myeloid progenitors from three healthy donors (A, B and C) were infected with lentiviral shRNA constructs targeting RINF expression (shRNA/RINF-3 and -4) or control vectors (empty and shRNA/scramble)-and treated with cytokines (IL3 and G-CSF at 20 ng/mL, SCF at 50 ng/mL) to drive them into granulocytes. For each donor, myeloid differentiation was evaluated every two-three days of culture by cell morphology analysis after cytospins and MGG staining (scale bar, 25 µm). Cell differentiation of cultured progenitors developed with different kinetics. The figure shows morphology of the cell populations after 14, 30, and 18 days, respectively. Note that cell cultures infected with shRNA-RINF-3 or shRNA-RINF-4 constructs display more immature cells at the promyelocytic/myelocytic stage (indicated by arrows) than the controls (cell cultures infected with empty or scramble vectors). For donor A, the kinetic of granulocytic differentiation was fast (until day 10) and only a few adherent monocytes/macrophages persisted in control cultures at day 14.

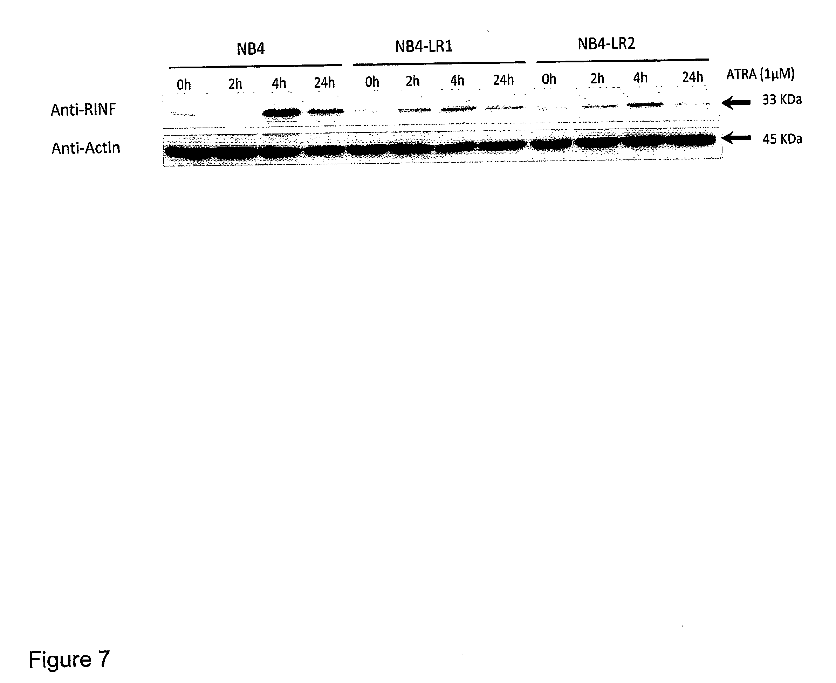

Figure 7 shows the expression of RINF protein in total extracts from NB4, NB4-LR1 and NB4-LR2 cells treated with ATRA (1 µM). RINF was detected with our customized polyclonal rabbit antibody (see Methods) that reveals a specific band at 33 kDa. Actin was used as a loading control. RINF expression was more pronounced in NB4 cells, than in the two resistant subclones NB4-LR1 and NB4-LR2.

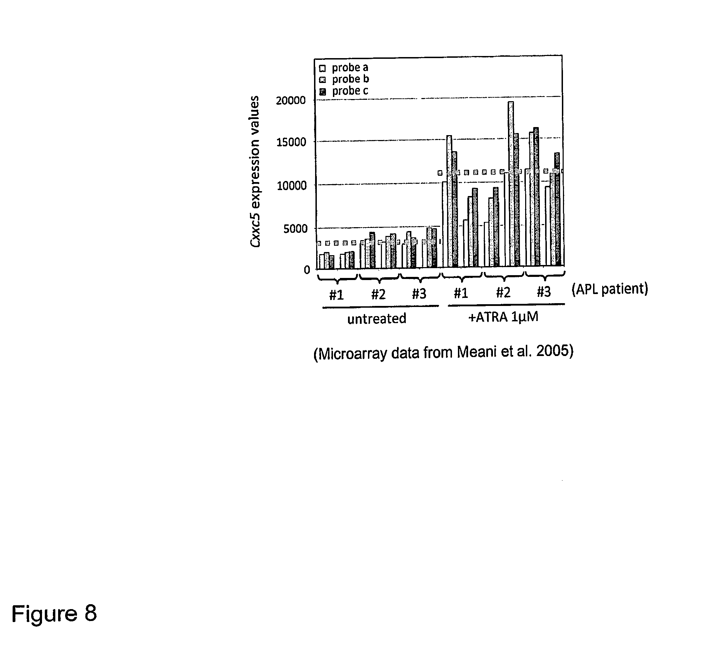

Figure 8 shows the *Cxxc5* mRNA expression in blasts derived from three (PML-RARa positive) APL patients. Cells were treated or not with ATRA (1 µM) during 4h. Data have been extracted from the publically available database Arrayexpress (E-MEXP-149). Microarray experiments were performed by Meani et *al.* according to Affymetrix GeneChip Human Genome HG-U133A and HG-U133B (2 represented hybridizations for each sample). The quantitation type used for the expression value measurement is *affymetrix:CHPSignal.* The 3 probes targeting *Cxxc5* expression were probe a (222996_s_at), b(224516_s_at) and c (233955_x_at). The two dotted lines indicate untreated and ATRA-treated group means. We applied the Paired-student's t-Test to show that the values for these two groups were significantly different (p-value < 0.05).

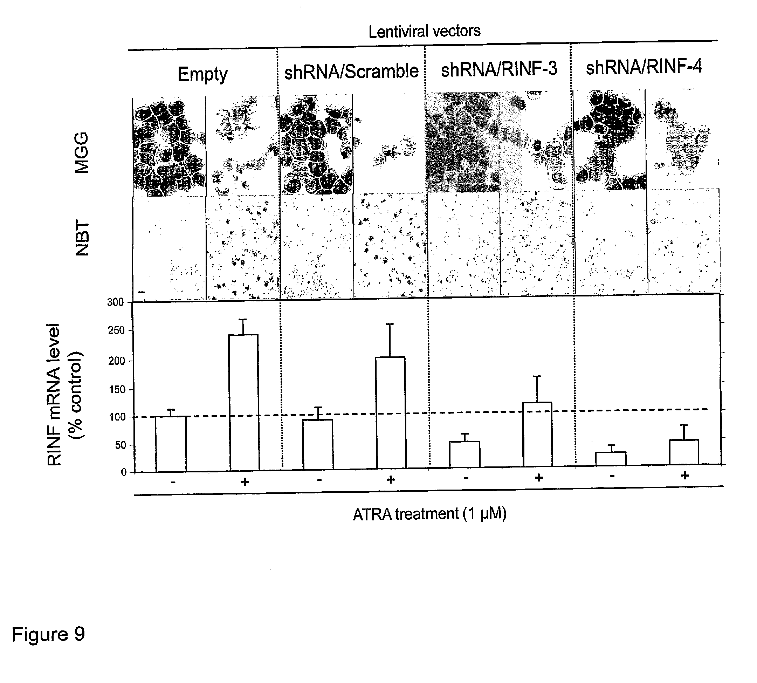

Figure 9 shows that shRNA-mediated silencing of *Rinf* delays ATRA-induced terminal differentiation of HL60 cells. HL60 cells were infected with Empty, shRNA/Scramble, shRNA/RINF-3 or shRNA/RINF-4 lentiviral vectors and selected. Cells were then treated or not with ATRA (1µM). *RINF* mRNA expression was assessed by quantitative RT-PCR (% of untreated mock-control at 6 hours of culture). Terminal differentiation was assessed by the NBT reduction assay at day 2 and by cell morphology at day 6 and (scale bars, 25 µm).

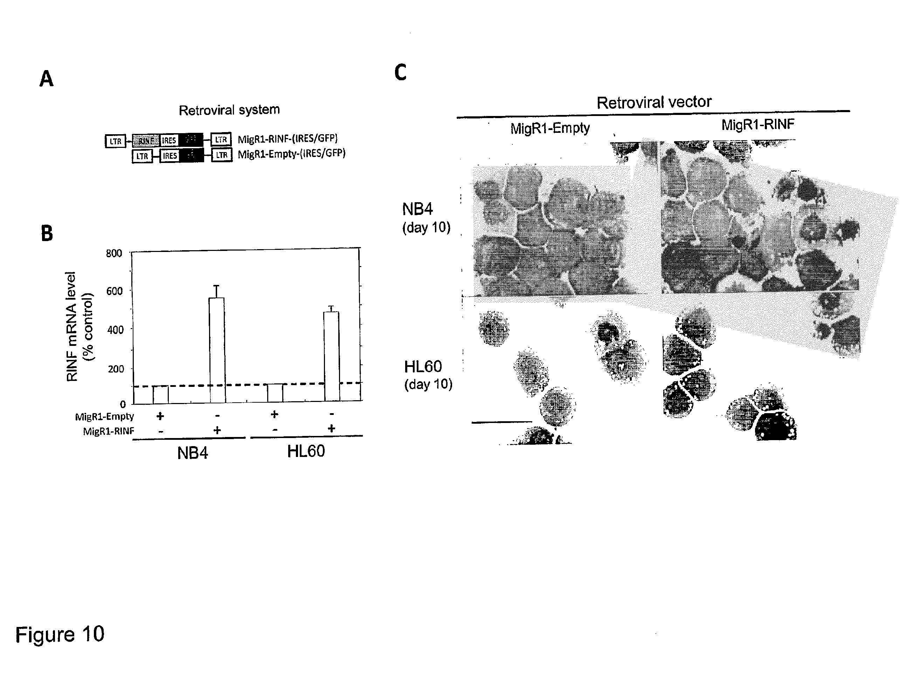

Figure 10 shows that RINF over-expression is not sufficient to induce differentiation of NB4 and HL60 cell lines. (A) A retroviral system derived from Murine Stem Cell Virus (MSCV) was used to over-express RINF in the two cell lines. The cells were infected and sorted 9 days post-infection for GFP expression. (B) For the two cell lines, RINF mRNA expression was measured (here at day 10) by quantitative RT-PCR and represented in % of their respective mock control (+/- s.e.m). (C) Cells were cytospined and stained with MGG for cell morphology analysis here visualized at two different magnitudes (scale bars, 25 µm). No sign of differentiation was noticed even after 10 days of RINF over-expression. A few percentage of cell death (about 10% of the total) was consistently noticed in cell cultures over-expressing RINF.

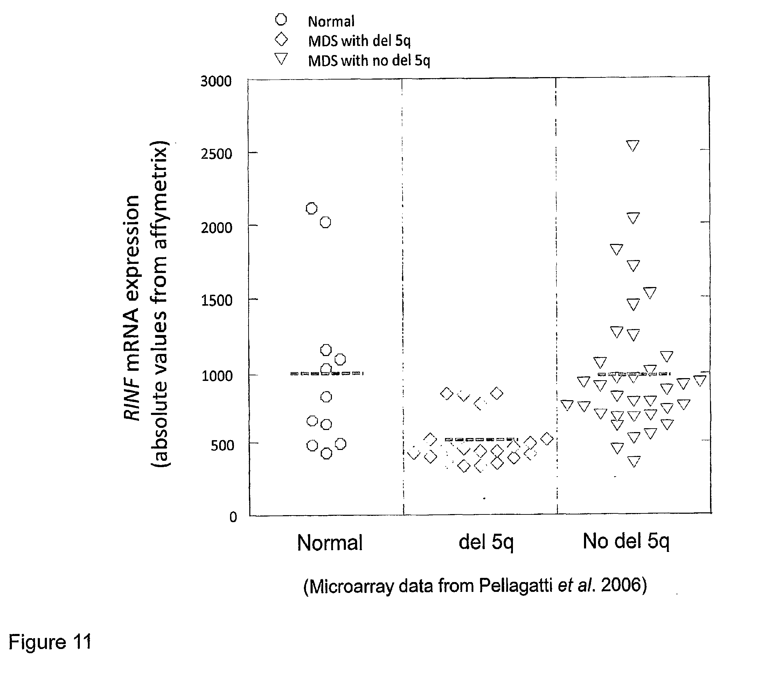

Figure 11 shows Cxxc5 mRNA expression in CD34+ cells derived from 55 patients suffering from myelodysplasia (MDS). Data have been extracted from the publicly available databases Arrayexpress (E-GEO-4619) and Gene Expression Omnibus (GDS2118). Microarray experiments were performed by Pellagatti et *al.* according to GeneChip Human Genome U133 Plus 2.0 arrays (Affymetrix). Each point represents 1 patient with MDS or a healthy individual. The three dotted lines indicate group means (998.1, 512.2 and 985.9) for normal healthy donors (n=11), MDS with deletion 5q (n=20) or MDS without del 5q (n=35). The values for Normal and MDS with del(5q) were significantly different according to the Wilcoxon two sample test (p<0.003). URLs: http://www.ebi.ac.uk/arrayexpress/ (ArrayExpress) and http://www.ncbi.nlm.nih.gov/geo/ (Gene Expression Omnibus)
Figure 12 shows RINF mRNA expression detected by RQ-PCR in bone marrow cells from patients with various hemapathies

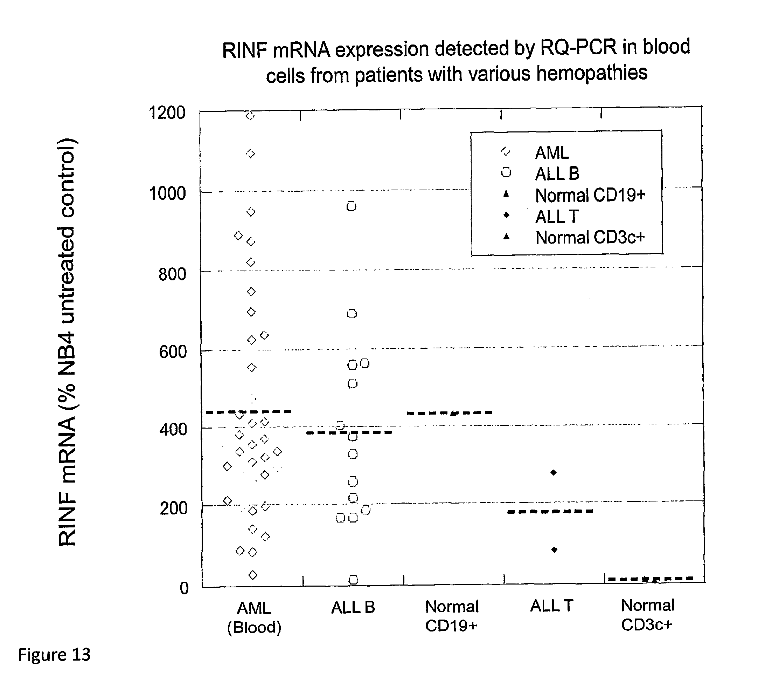

Figure 13 shows mRNA expression detected by RQ-PCR in blood cells from patients with various hemopathies.

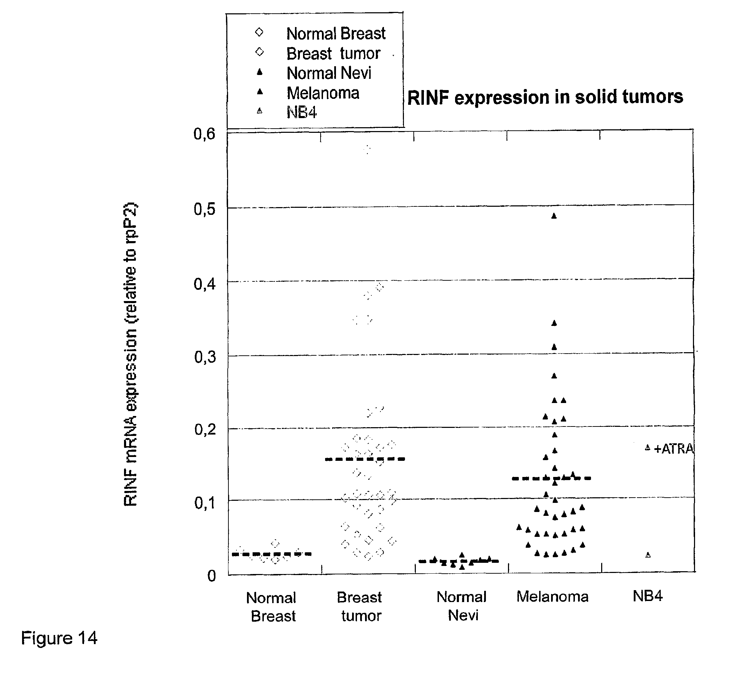

Figure 14 shows RINF expression in solid tumors

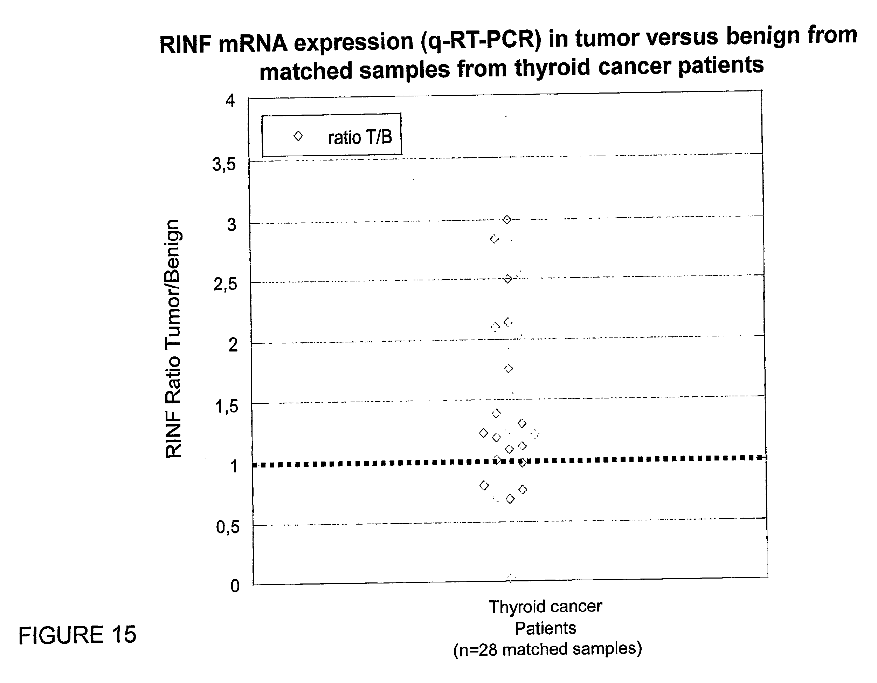

Figure 15 shows RINF expression in tumor vs benign from matched samples from thyroid cancer patients.

### Experimental results

### Identification of Cxxc5, a gene early induced by retinoic acid in the NB4 cell line

In our search for new target genes of retinoic acid in APL, we performed microarray experiments using NB4 cells treated during short periods of time (90 minutes and 3 hours) with ATRA (1 µM). Microarray expression analyzes allowed us to identify 35 genes consistently up-regulated at 90 minutes of treatment, with at least a two-fold induction compared to untreated control, and that remained up-regulated after 3 hours (Table 1). Upon careful analysis and classification of these 35 genes, nine genes encoding well known or putative transcription factors were identified. The other 26 genes did not meet the criteria for being putative transcription factors according to gene ontology and/or sequence analysis. Seven out of the nine candidate genes encode well characterized transcription factors, known to be expressed in the myeloid tissue during granulocytic or monocytic differentiation. Importantly, six of these genes have previously been identified as early induced by retinoic acid in NB4 cells, five being direct targets. These observations confirmed and validated the accuracy of our microarray screen.

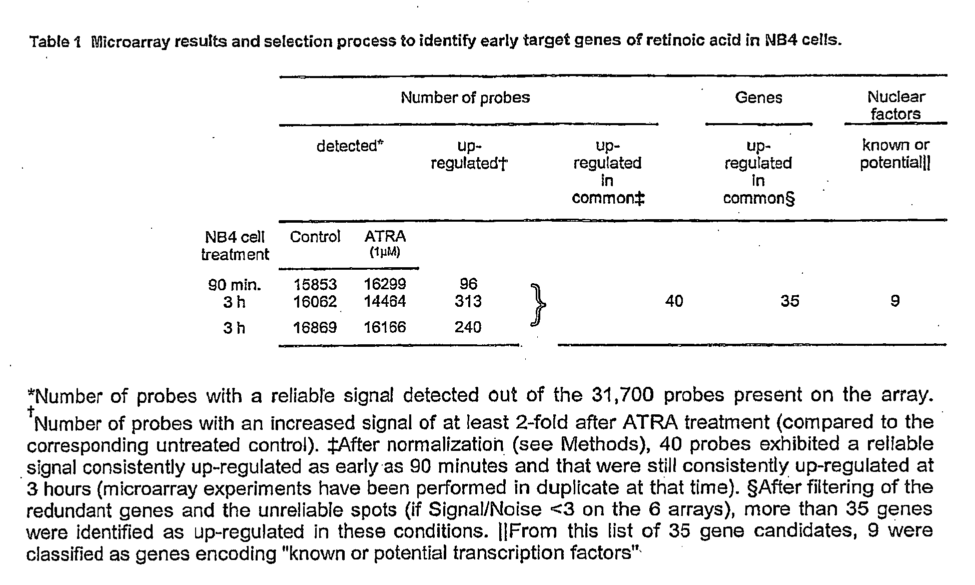

This relates to Cxxc5, a genomic sequence so far not subjected to any functional characterisation.

Our choice was made after two decisive experimental findings. First, quantitative RT- PCR analysis (Figure 1A) exquisitely confirmed ATRA-induction of Cxxc5 mRNA expression (here observed at 4 hours of retinoic acid treatment). Second, pre- treatment of NB4 cells with the protein synthesis inhibitor cycloheximide, 30 minutes prior to retinoid addition and continued for 4 hours, did not block the CXXC5 mRNA up-regulation by ATRA (Figure 1A). These experiments indicate that ATRA-mediated transcription of Cxxc5 does not require de novo protein synthesis and suggest that Cxxc5 is a primary target of retinoic acid. This has been confirmed by ChIP experiments (Figure 1 B) that demonstrated a direct binding of retinoid receptors (RAR and/orPML-RARα to the promoter of Cxxc5 gene in NB4 cells.

### In silico characterization of Cxxc5 gene

The main genomic features of Cxxc5 (ENSG00000171604) available from data bases are briefly summarised in Figure 2. The *Cxxc5* gene is located on the long arm of chromosome 5, at 5q31.3 (Figure 2A). The gene spans 35.5 kbps and is organized in 4 exons (Figure 2B). The upstream promoter region of this gene has not yet been functionally analyzed, but relevant for our study, it contains a potential retinoid-responsive element at -3116 bps from the transcription start site. Despite the existence of two potential alternative transcription start sites, one in exon 1 and the other in exon 2, the two mRNA sequences reveals the same open reading frame with the start codon in exon 3. Conceptual translation predicts a protein of 322 amino acids (Figure 2C) and a theoretical molecular weight of 32.98 kDa . Protein sequence analysis revealed a unique conserved domain, a typical CXXC zinc finger motif (*Cx₂Cx₂Cx₄₋₅Cx₂Cx₂C₉₋₁₅Cx₂Cx₄C*) between amino acids 257 and 302, in proximity to the C-terminal end. This motif known to contain eight conserved cysteine residues coordinating two zinc ions is found in eleven other proteins (often chromatin-associated proteins) and is assumed to recognize unmethylated CpG. Figure 2D presents an alignment of all known members of the protein family bearing this motif.

### Gene expression profile of Cxxc5 in promyelocytic cells treated with retinoids

In agreement with our microarray data, time-course analysis of CXXC5 mRNA levels (by quantitative RT-PCR) demonstrated a two-fold induction as early as 90 minutes of ATRA treatment (Figure 3A). The transcript level of *Cxxc5* reached its highest level at 12 hours of treatment (approximately six-fold the expression level observed in untreated control). Then, its level remained stably up-regulated for at least 48 hours (Figure 3A). To corroborate *Cxxc5* expression and induction at the protein level, we customized a polyclonal antibody directed against amino acids 45-58 of the predicted CXXC5 polypeptide chain (see Methods). Basal expression level of CXXC5 protein was very low in untreated NB4 cell extracts (Figure 3B) but a strong band, specific to CXXC5 protein (verified by competition with the immunogenic peptide, not shown), appeared at the expected molecular weight of 33 kDa within 4 hours after onset of ATRA treatment. The kinetic of induction of the CXXC5 protein is in agreement with the induction observed at the mRNA level. Interestingly, compared to NB4 cells, only a very weak induction of CXXC5 protein was noticed following ATRA treatment of the maturation resistant subclones NB4-LR1 and NB4-LR2 (Figure 7). These observations were compatible with a potential participation of CXXC5 during terminal maturation of APL cells. Moreover, data extracted from publicly available microarray datasets (Figure 8), confirmed the early induction of *Cxxc5* mRNA by ATRA in primary cells from APL patients, validating the data obtained with the NB4 cell line.

### Subcellular localisation of Retinoid-Inducible Nuclear Factor, RINF (CXXC5)

Western-blot analysis of nuclear and cytoplasmic fractions of ATRA-treated NB4 cells clearly detected expression of CXXC5 in the nuclear extracts (Figure 3C). This increased expression level lasted at least for 48 hours. Because our polyclonal antibody barely detected the relevant epitope (amino acids 45-58) of the folded native protein, we generated a plasmid encoding a FLAG-tagged CXXC5 for *in situ* immunochemistry experiments. *In situ* confocal analysis of MCF7 cells transiently transfected with this construct showed a strong nuclear staining (Figure 3D) and confirmed the nuclear localisation pattern of CXXC5 protein. Of note, from cell to cell and according to the level of over-expression, the fluorescence pattern was partly associated to a fine chromatin matrix, nucleoplasm and/or discrete punctuated structures in the nucleus. However, we did not detect any fluorescent signal at the nuclear membrane or in the nucleoli.

Based on these experimental evidences, we propose to rename CXXC5 as RINF, for Retinoid-Inducible Nuclear Factor. In keeping with this proposed name, a detailed analysis of the RINF (CXXC5) sequence revealed a putative Nuclear Localization Signal (NLS) between amino acid residues 257 and 262 (KKKRKR), located to the N-terminal basis of the zinc finger domain.

### Expression patterns of RINF in different myeloid cell lines and various human tissues

We have also evaluated basal expression levels of the *Rinf* gene in other myeloid cell lines (Figure 3E-F) and in various human tissues (Figure 3G) as well as its potential regulation by retinoids. The more immature cell lines tested, K562 and LAMA-84 cells showed the highest *RINF* mRNA levels (16- and 12-fold the basal level of NB4 cells respectively) and also the highest RINF protein expression. Neither *RINF* mRNA nor protein levels seemed up-regulated upon ATRA treatment in these cell lines (Figure 3E-F). In comparison, the HL60 (M2-subtype) and NB4 (M3-subtype) cell lines both harbour low basal levels of *RINF* mRNA and protein. Importantly, treatment of the latter cell lines with ATRA indicated that HL60 cells (known to embark on terminal differentiation upon such a treatment) behave similarly to NB4 cells with respect to RINF induction (here observed at 4 hours), both at the mRNA and protein level. Importantly, this induction in HL60, a cell line lacking the expression of the chimeric receptor PML-RARα demonstrates that PML-RARα is not required for RINF induction by ATRA. In agreement with this observation, we noticed that ATRA also induces *RINF* mRNA expression in two other cell lines with a basal expression level similar to NB4 cells (data not shown), the A549 lung carcinoma (a three-fold increase) and the HeLa cervix cancer cells (a two-fold increase). Finally, to substantiate the fact that RINF expression was not restricted to myeloid tissue, we performed a western-blot analysis on various protein extracts from different organs (Figure 3G). RINF protein showed different expression levels in the human tissues tested, the highest expression level was in placenta and the lowest in brain. Altogether, these data indicate that the regulated expression and the putative function of RINF is not dependent on PML-RARα expression and encompass a field of interest much broader than the APL pathology.

### RINF expression is required for differentiation of promyelocytic leukemia cells

In order to investigate the potential involvement of RINF in terminal differentiation of NB4 cells following ATRA-treatment, we performed a RNA-interference approach using shRNAs delivered by lentiviral vectors (Figure 4A). After infection and selection of NB4 cells stably expressing each of the 5 different shRNA constructs directed against the *RINF* mRNA, their efficiency (to target basal *RINF* mRNA expression level) was compared to control vectors, one expressing scrambled shRNA and one being the empty vector. The two best knock-down efficiencies were observed with shRNA/RINF-3 (61%) and shRNA/RINF-4 (85%) with which no proliferation changes or morphological alterations were noticed in NB4 cells in the absence of retinoid. With such a silencing efficiency (Figure 4A), we could reasonably expect to efficiently block *RINF* mRNA induction by ATRA in an important proportion of the cells, and in this manner, evaluate the consequence on the cell phenotype following ATRA treatment.

Strikingly, expression of these two shRNA constructs gives the NB4 cell population the capacity to proliferate in the presence of pharmacological doses of ATRA (Figure 4B). Indeed, while the cells expressing control vectors ineluctably differentiated and died within a week upon ATRA treatment, both cell cultures infected with either shRNA/RINF-4 or shRNA/RINF-3 lentiviral vectors bypassed the growth arrest (Figure 4B) and the terminal differentiation process (Figure 4C). The efficiency of our RNA interference strategy to block ATRA-induced *RINF* mRNA expression was evaluated by quantitative RT-PCR (Figure 4C) at 12 hours of treatment, a time at which *RINF* mRNA expression was maximum (see also Figure 3A). Importantly, knockdown levels obtained with shRNA/RINF-3 (at about 50%) and shRNA/RINF-4 (60%) compared to control vectors treated with ATRA, were enough to rescue approximately 30%-50% of NB4 cells from terminal differentiation as assessed by reduction of NBT and morphological analysis (Figure 4C, left panel).

Of note, these cells (shRNA/RINF-3 and -4) continued to proliferate in the presence of retinoids and, even after two weeks of culture without ATRA (from day 6 to 20), their resistance to ATRA was confirmed, and remained closely associated to an efficient block in *RINF* mRNA expression (Figure 4C, right panel). Altogether, these results demonstrate that shRNA-mediated *RINF* knockdown impairs ATRA-induced terminal differentiation of promyelocytic leukemia cells and strongly suggest that RINF expression is required for retinoid-induced terminal maturation of NB4 cells.

RINF contribution was then evaluated in the HL60 cell line committed into the granulocytic differentiation pathway with pharmacological doses of ATRA (Figure 9). As observed with NB4 cells, the two shRNAs efficiently target RINF expression (Figure 9) and delayed the maturation process assessed by NBT and morphological analysis, underpinning the functional involvement of RINF during granulocytic differentiation. However, in contrast to NB4-shRNA/RINF cells that continued to grow in the presence of retinoids, ATRA-treated HL60-shRNA/RINF cells inevitably declined and died within 12 days.

Finally, we wondered if RINF over-expression would be sufficient to induce granulocytic maturation of NB4 or HL60 cells in the absence of ATRA (Figure 10). Our data indicate that ectopic RINF expression (Figure 10 B) is necessary but not sufficient to trigger granulocytic differentiation of leukemic cells. Indeed, even after 10 days of RINF over-expression, we did not notice any sign of differentiation neither at the morphological level (Figure 10, panel C), nor at the molecular level (using anti-CD11 b surface marker, data not shown).

### RINF expression and function during cytokine-driven myelopoiesis of normal CD34+ progenitor cells

Our above findings clearly indicate a broad spectrum of RINF expression in hematopoietic and other human tissues. These data indicated that although inducible by pharmacological concentrations of ATRA and required for differentiation of some myeloid leukemia cells, RINF expression may well be regulated physiologically by cytokines during normal progenitor myelopoiesis. In this case, its regulated expression could represent a more general event also occurring during normal development along the granulocytic lineage.

To test this hypothesis, we examined *RINF* expression during cytokine-induced granulocytic differentiation of CD34+ cells isolated from bone marrow of a healthy adult donor. Differentiation was assessed by morphological changes (Figure5A) and analysis of *CD34, CD11b* and G-CSFR expression comparatively to *RINF* (Figure 5B). The time-course expression profile of *RINF* mRNA levels was determined by quantitative RT-PCR analysis. After an initial decrease (from day 2 to 4), the *RINF* mRNA level reached its minimal expression between day 4 and 6, a time at which most of the cells in culture were at the blastic and promyelocytic stage. Later on, between day 6 and 8, *RINF* mRNA expression increased approximately 3.5-fold from its minimum level, concomitantly to terminal cell maturation into myelocytes, metamyelocytes, and ultimately into short-lived polynuclear neutrophiles (Figure 5A-B). The detection of *RINF* mRNA in CD34+ progenitors and in normal hematopoietic cells during cytokine-driven differentiation confirms that its expression is not restricted to APL cells (and is therefore not dependent on PML-RARα expression) and most importantly that its induction is not restricted to ATRA pharmacological signaling. These data are consistent with its potential role during normal myelopoiesis.

In order to evaluate the functional relevance of RINF expression during normal myelopoiesis, CD34+ progenitor cells directed toward the granulocytic lineage by cytokine treatment were infected with the lentiviral shRNA constructs to knock down RINF expression. Like non-infected cells, most of the cells infected with control vectors (shRNA/scramble and mock control) matured into polynuclear granulocytes (Figures 5A and 6) before dying and progressively disintegrated in cell cultures (not shown). In the three cell cultures tested from different donors, granulocytic differentiation occurred with different kinetics (see Figure 6), all cultures ending with cell maturation in polynuclear neutrophiles that rapidly died. Only few monocytes and plastic-adherent macrophages, known to have a much longer life, persisted in the cultures. Importantly, cell populations infected under the same conditions with shRNA/RINF-3 and shRNA/RINF-4 (Figure 6) showed significant accumulation of un-matured cells (promyelocytes and myelocytes). This difference in granulocytic maturation, observed with the two shRNAs compared to the control cultures, exquisitely mirrored the results obtained with the ATRA treated leukemic cell lines NB4 and HL60 and suggests a similar role of RINF during normal myelopoiesis.

### Discussion

The multistage process of hemopoietic cell differentiation has long served as a model study for the understanding of tumor etiology and for the design of therapeutic strategies. Disturbances in the developmental programs that rule the production of mature functional cells frequently result from genetic or epigenetic alterations (gene deletions, mutations, methylation, etc..) in a limited number of key regulatory genes, whose functions of predilection are signal transduction and/or gene transcription control. In the particular case of hemopoietic malignancies, the last decade has brought major advances in the finding of these key regulatory genes but uncertainty remains on their functional hierarchy.

In the present study, we have identified and characterized a novel member of the zinc-finger family, CXXC5 (RINF), and shown its implication during retinoid-induced terminal differentiation of myelocytic leukemia cells, but also during cytokine-driven normal myelopoiesis. Zinc-finger-containing proteins constitute one of the largest protein superfamilies in the mammalian genome and can be classified into evolutionary and functionally divergent protein families, with structurally different conserved domains interacting with DNA, RNA, lipids, or other proteins. The CXXC-type zinc finger is found in a small subset of proteins (Figure 2) involved in chromatin remodelling through their histone methyl-transferase (MLL, MLL2), histone demethylase (FBXL-10, -11, -19), DNA-methyl-transferase (DNMT1), orCpG-binding (CGBP, MBD1) activities. The CXXC domains of MLL, CGBP, and MBD1 have been shown to bind to non-methylated CpG and the conserved KFGG motif (in the second linker of the zinc finger) is essential for this recognition. The paralogs lacking the KFGG motif (CXXC-4, -5, -6, and -10, Figure 2) are expected not to share the latter property and one of the future challenges will be to identify the molecular target(s) recognized by this specific subset of proteins with conserved CXXC domains but lacking the KFGG motif. Interestingly, three members of the CXXC-type family (containing or not the KFGG-motif), *MLL, MLL2,* and *LCX,* have previously been involved in leukemogenesis through chromosomal translocations or internal rearrangements. *MLL* is one of the most frequently translocated genes in leukemia and in spite of more than 30 different fusion partners that have been described, the CXXC domain is retained in all known MLL fusion proteins and the domain appears to be essential for myeloid transformation, underpinning the biological importance of this zinc finger sub-type even if the mechanism of action remains to be clarified.

The expression and the induction patterns of the *Cxxc5* (*Rinf*) gene reported here supported a functional involvement of RINF in both normal and tumoral myelopoiesis, at least in the latest stages of the maturation process (promyelocyte, myelocyte). Knock-down experiments, using specific RNAi targeting, further demonstrated that *RINF* expression was necessary for the terminal differentiation process of promyelocytic leukemia cells. However, and importantly, our study clearly supports the idea that *Rinf* pathophysiology is not restricted to APL, but may have broader implication in other hemopoietic malignancies and normal hemopoiesis. Thus, the *Rinf* status (such as mutations, aberrant expression/induction) may have predictive value for ATRA-responsiveness, and therefore being an important tool for decision-making on therapeutic regimens for AML patients.

In the clinical context, an exciting prospect concerning *Cxxc5* (*Rinf*) biology will be to evaluate if its de-regulated expression could contribute to the etiology of some hematological diseases. Indeed, interstitial deletion or complete loss of the long arm of chromosome 5 are recurrent chromosome abnormalities in malignant myeloid disorders characterized by abnormal myelopoiesis and an excess of blasts in the bone marrow, like myelodysplasia (MDS, often described as a preleukemic disorder) and acute myeloid leukemia (AML). Despite extensive studies that have delineated the major commonly deleted region (CDR) to chromosome band 5q31, candidate tumor suppressor genes presumed to contribute to leukemogenesis remain to be identified in this region of 4 Mbp in size. It is important to note that, inside this segment, *Rinf* localises less than 20 kbp from the distal marker D5S594 that delineate the smallest (less than 1 Mbp) CDR described at his date. Surprisingly, the *Rinf* gene has so far escaped genetic and functional investigation, probably because the gene has been considered to localize outside the CDR. Even so, the close proximity of *Rinf* to the CDR may affect *Rinf* expression due to loss of regulatory sequences upstream of the identified gene, as well as more distant deletions within 5q31. Our findings combined with data in the literature led us to consider *Rinf* to be a strong candidate for a tumor suppressor gene of myeloid cell transformation. Interestingly, mining publicly available microarray databases for myeloid pathologies, we brought to light a lower *RINF* mRNA expression in CD34+ cells from myelodysplastic patients with deletion 5q compared to normal healthy donors (Figure 11). This observation is compatible with a putative haploinsufficiency mechanism and it will be important to evaluate the genuine contribution of *rinf* deregulation in these hemopathies, as it has been recently described for the ribosomal gene *rpS14* (located at 5q32 in a distinct CDR) in another subgroup of MDS with refractory anemia, the 5q-syndrome.

Since *Rinf* expression is not restricted to myeloid tissue, this gene may also be involved in development and/or homeostasis of other tissues. Its direct induction by retinoids, which does not require *de novo* synthesis of an intermediate protein regulator, suggests that *Rinf* might mediate, at least in part, some of the pleiotropic effects of retinoids, for instance such as their anti-proliferative action in various solid tumors, even independently of any differentiation. Taken together, our findings support the hypothesis that *Rinf* expression, pharmacologically inducible by retinoids in different tissues, may have a broad interest because of its likely implication in several developmental processes and pathologies.

### SEQ ID NO 1 - 5 274097 295972

### Organization Applicant

Street :
City :
State :
Country :
PostalCode :
PhoneNumber :
FaxNumber :
EmailAddress :

<110> OrganizationName : Bergen Teknologioverføring AS

### Application Project

<120> Title : Novel retinoid inducible factor, and uses thereof
<130> AppFileReference : P11828NO01
<140> CurrentAppNumber :
<141> CurrentFilingDate : __-_-_

### Sequence

<213> OrganismName : Homo sapiens
<400> PresequenceString : 
gttttgccgg cagcccccgg gcagcgttca tagctcctgc ccgggcgggc gcgcggcggc 60
ggcggcagag gcggctgagc ctgagcgggg atgtagaggc ggcggcagca gaggcggcac 120
tggcggcaag agcagacgcc cgagccgagc gagaagagcg gcagagcctt atcccctgaa 180
gccgggcccc gcgtcccagc cctgcccagc ccgcgcccag ccatgcgcgc cgcctgctga 240
gtccgggcgc cgcacgctga gccctccgcc cgcgagccgc gctcagctcg ggggtgatta 300
gttgcttttt gttgtttttt aatttgggcc gcggggaggg ggaggagggg caggtgctgc 360
aggctccccc ccctccccgc ctcgggccag ccgcggcggc gcgactcggg ctccggaccc 420
gggcactgct ggcggctgga gcggagcgca ccgcggcggt ggtgcccaga gcggagcgca 480
gctccctgcc ccgcccctcc ccctcggcct cgcggcgacg gcggcggtgg cggcttggac 540
gactcggaga gccggttcgg tgcgcggccg gggccggagt tcgctgcaag tcggcggaaa 600
gtttggctgc gcgggttccc ccgaagttca gagtgaagac atttccacct ggacacctga 660
ccatgtgcct gccctgagca gcgaggccca ccaggcatct ctgttgtggg cagcagggcc 720
aggtcctggt ctgtggaccc tcggcagttg gcaggctccc tctgcagtgg ggtctgggcc 780
tcggccccac catgtcgagc ctcggcggtg gctcccagga tgccggcggc agtagcagca 840
gcagcaccaa tggcagcggt ggcagtggca gcagtggccc aaaggcagga gcagcagaca 900
agagtgcagt ggtggctgcc gccgcaccag cctcagtggc agatgacaca ccaccccccg 960
agcgtcggaa caagagcggt atcatcagtg agcccctcaa caagagcctg cgccgctccc 1020
gcccgctctc ccactactct tcttttggca gcagtggtgg tagtggcggt ggcagcatga 1080
tgggcggaga gtctgctgac aaggccactg cggctgcagc cgctgcctcc ctgttggcca 1140
atgggcatga cctggcggcg gccatggcgg tggacaaaag caaccctacc tcaaagcaca 1200
aaagtggtgc tgtggccagc ctgctgagca aggcagagcg ggccacggag ctggcagccg 1260
agggacagct gacgctgcag cagtttgcgc agtccacaga gatgctgaag cgcgtggtgc 1320
aggagcatct cccgctgatg agcgaggcgg gtgctggcct gcctgacatg gaggctgtgg 1380
 
caggtgccga agccctcaat ggccagtccg acttccccta cctgggcgct ttccccatca 1440
acccaggcct cttcattatg accccggcag gtgtgttcct ggccgagagc gcgctgcaca 1500
tggcgggcct ggctgagtac cccatgcagg gagagctggc ctctgccatc agctccggca 1560
agaagaagcg gaaacgctgc ggcatgtgcg cgccctgccg gcggcgcatc aactgcgagc 1620
agtgcagcag ttgtaggaat cgaaagactg gccatcagat ttgcaaattc agaaaatgtg 1680
aggaactcaa aaagaagcct tccgctgctc tggagaaggt gatgcttccg acgggagccg 1740
ccttccggtg gtttcagtga cggcggcgga acccaaagct gccctctccg tgcaatgtca 1800
ctgctcgtgt ggtctccagc aagggattcg ggcgaagaca aacggatgca cccgtcttta 1860
gaaccaaaaa tattctctca cagatttcat tcctgttttt atatatatat tttttgttgt 1920
cgttttaaca tctccacgtc cctagcataa aaagaaaaag aaaaaaattt aaactgcttt 1980
ttcggaagaa caacaacaaa aaagaggtaa agacgaatct ataaagtacc gagacttcct 2040
gggcaaagaa tggacaatca gtttccttcc tgtgtcgatg tcgatgttgt ctgtgcagga 2100
gatgcagttt ttgtgtagag aatgtaaatt ttctgtaacc ttttgaaatc tagttactaa 2160
taagcactac tgtaatttag cacagtttaa ctccaccctc atttaaactt cctttgattc 2220
tttccgacca tgaaatagtg catagtttgc ctggagaatc cactcacgtt cataaagaga 2280
atgttgatgg cgccgtgtag aagccgctct gtatccatcc acgcgtgcag agctgccagc 2340
agggagctca cagaagggga gggagcacca ggccagctga gctgcaccca cagtcccgag 2400
actgggatcc cccaccccaa cagtgatttt ggaaaaaaaa atgaaagttc tgttcgttta 2460
tccattgcga tctggggagc cccatctcga tatttccaat cctggctact tttcttagag 2520
aaaataagtc ctttttttct ggccttgcta atggcaacag aagaaagggc ttctttgcgt 2580
ggtcccctgc tggtgggggt gggtccccag ggggccccct gcggcctggg cccccctgcc 2640
cacggccagc ttcctgctga tgaacatgct gtttgtattg ttttaggaaa ccaggctgtt 2700
ttgtgaataa aacgaatgca tgtttgtgtc acgaaaaaaa aaaaaaaaaa aaaaaaaaaa 2760
aaaaaaaaaa aaaaaaaa 2778
<212> Type : DNA
<211> Length : 2778
   SequenceName : rinf1
   SequenceDescription :

### sequence

<213> OrganismName : Homo sapiens
<400> PreSequenceString : 
atgtcgagcc tcggcggtgg ctcccaggat gccggcggca gtagcagcag cagcaccaat 60
ggcagcggtg gcagtggcag cagtggccca aaggcaggag cagcagacaa gagtgcagtg 120
gtggctgccg ccgcaccagc ctcagtggca gatgacacac caccccccga gcgtcggaac 180
aagagcggta tcatcagtga gcccctcaac aagagcctgc gccgctcccg cccgctctcc 240
cactactctt cttttggcag cagtggtggt agtggcggtg gcagcatgat gggcggagag 300
tctgctgaca aggccactgc ggctgcagcc gctgcctccc tgttggccaa tgggcatgac 360
 
ctggcggcgg ccatggcggt ggacaaaagc aaccctacct caaagcacaa aagtggtgct 420
gtggccagcc tgctgagcaa ggcagagcgg gccacggagc tggcagccga gggacagctg 480
acgctgcagc agtttgcgca gtccacagag atgctgaagc gcgtggtgca ggagcatctc 540
ccgctgatga gcgaggcggg tgctggcctg cctgacatgg aggctgtggc aggtgccgaa 600
gccctcaatg gccagtccga cttcccctac ctgggcgctt tccccatcaa cccaggcctc 660
ttcattatga ccccggcagg tgtgttcctg gccgagagcg cgctgcacat ggcgggcctg 720
gctgagtacc ccatgcaggg agagctggcc tctgccatca gctccggcaa gaagaagcgg 780
aaacgctgcg gcatgtgcgc gccctgccgg cggcgcatca actgcgagca gtgcagcagt 840
tgtaggaatc gaaagactgg ccatcagatt tgcaaattca gaaaatgtga ggaactcaaa 900
aagaagcctt ccgctgctct ggagaaggtg atgcttccga cgggagccgc cttccggtgg 960
tttcagtga 969
<212> Type : DNA
<211> Length : 969
   SequenceName : rinf2
   SequenceDescription :

### sequence

<213> OrganismName : Homo sapiens
<400> PreSequenceString : 
MSSLGGGSQD AGGSSSSSTN GSGGSGSSGP KAGAADKSAV VAAAAPASVA DDTPPPERRN 60
KSGIISEPLN KSLRRSRPLS HYSSFGSSGG SGGGSMMGGE SADKATAAAA AASLLANGHD 120
LAAAMAVDKS NPTSKHKSGA VASLLSKAER ATELAAEGQL TLQQFAQSTE MLKRVVQEHL 180
PLMSEAGAGL PDMEAVAGAE ALNGQSDFPY LGAFPINPGL FIMTPAGVFL AESALHMAGL 240
AEYPMQGELA SAISSGKKKR KRCGMCAPCR RRINCEQCSS CRNRKTGHQI CKFRKCEELK 300
KKPSAALEKV MLPTGAAFRW FQ 322
<212> Type : PRT
<211> Length : 322
   SequenceName : RINF
   SequenceDescription :

### Sequence

<213> OrganismName : Homo sapiens
<400> PreSequenceString :
   cgagttcatg aggtgagc 18
<212> Type : DNA
<211> Length : 18
   SequenceName : RARE
   SequenceDescription :

### Sequence

<213> OrganismName : Homo sapiens
<400> PreSequenceString : 
ccccaagcac tgtctgagca ccccattcct tggagtctga agggaagggc agagtagggg 60
gtggtagctg gggtcttggg gccccaggtc tgaagagaaa gaggctgggc cattttcgga 120
agtctcagtg cgggccattt ctgaccctgc actgccctcc aggaccagat ttgtagagag 180
gaggctgagc cccaccctgc ctcctccagc aggcctcccc cacaccacca caccctagcc 240
 
ttcaggcttt gggtcgggca gggcactttc agggcaggct ggcttctcct gcccagggca 300
cccctcctcc aggtgcaggg ctccagattg ggacactgtg cctctgcctc ccctaaggcc 360
ccattcagtt acctccccac ctctcagcaa tgctctccct ccctcttcct gaagtgtggc 420
agggaggctg gagccacagc tttggcctct gggagcactc agtcccactc ccataggaca 480
gagttgaacc atgattgtgt ctagagaagc caagggttat tgtcaggggc acaacagccc 540
ccaaactgtc cctctcccca tcaatatccc atatacaggc tgagttccct gtgcctcact 600
gagaacaggg ctgtatctgg ttgtctatgg cgtgtatgta gctgtgcctg ggcaatggga 660
aatatagctt gcttcctgct ttctctcagc ctctgtcccc agagtcattt tgttctggga 720
ctggagtctt ccttctgtcc tcatcccttc ccctctgttt acacaggtca aggccaggca 780
ttggccacca aactgacttc tcaggaatgg gcaagcctgg gttacttgag agacttggct 840
ttttgaattt tcatttcaca gctctctgct ccttcagacg tcatgggggt ggggtgggga 900
ggacctaaag tccagtggtc agtgcgtgag ggcccagacc caaaggcccg gggccacagt 960
ctggtgcggt taggctcctc cctgcctctg cccgtgtttg cacccccagg cctgccctgg 1020
gattttggct gaggtcacag tccagcatga ccccactgaa cactatcctg tgccctgccc 1080
cagcaggcag ggtttcaaat gcctttgctc ccatcctttg tgatacaggt gacagaactg 1140
gggcccaaag aagggtaaac cttagactga gttccaaatt ggccatcttt attggagatc 1200
ctaactccca cactcctctc catgtggcca tttccagtca ctggatgttt aatcaatgac 1260
agatattagc agataaatgg cagtcatgga aaatgcctca gacaaggcat ggattgaaat 1320
cctggttttg ctactttgta acctcacaca ggtgtccaag cctgtggatt taataagcct 1380
atgttcaaaa agcaattagc atggtccctg acaagcaaga gctcagaaag attctggcta 1440
caattgttat tactggtacc tctttggagg ccaagcttcg ggcccaaggc aggtagggga 1500
ctcctcctta ccttcaaaga attttcatgg cgggaggtgg tggcgagggg acaagtagtg 1560
agatgagcaa ataaaagcta tttctgtgca ttgtggtcac tgtggccata ggaggggtcc 1620
caaagccaga tgaaggaccc aaagccaggc cacagagtac atctgtcctg tgcatgcact 1680
gcaaaaaggc acctagcaaa gggagctagg aagctgaaag gcagtctgtg acctccttca 1740
tggtttcctg tatctgccaa aagtgggtac ctttttctga tttgcacaaa ggctagtggt 1800
ggccctgtag gtatggccca aactcaggct ggaagtggca gtctgagatg gttcccaggt 1860
gatatgtgct cctggtcttc catgaggagt tcatgaggtg agcttgatga ggcctatctt 1920
ggcagaggga atggtacatg caaagctctg gaaatctgga gaggagtctt ggggtgtcag 1980
agggaactgc cagtgttaga tggcattggt cagcagggcc tggcagggaa gagagtcagt 2040
tagggaagcc aggctgagta gggtagtctg tgaggaacca ctgcaggatg tgaagcagat 2100
gagcctcact ggaggctgtg gaaagcctcc ctagaatagc acatggcctc aggggtttga 2160
gaacagctct gatgccaatc tcatttcagt ttcctcatct gaataatggg gatgacatta 2220
ttgcaggggc tgctataagg gctaaataag ataatgtacc ttaactgttt agcacaatgc 2280
 
cctgctcagg gtaagggctg cacaaatacc agccatgggt attatcagca ggatccagga 2340
gagcacactc cagggtcccg ggtggacgca cccctcccta gaagacaagg ctggtgatgc 2400
aggggccatc tcccccagcg gtgctggaga ctgaaggagc ttcttcctga cttgaaggct 2460
gattggttca aaaaactcag caaaaagttt aatagcaagg tgagacttca attctccttt 2520
ttgaggtagg cagaggtggg agaatagtga accaagcttc ccttcttctt ggggaagggg 2580
gctgtggtgg ggacagtctc acccaagggc agagatcctc accccatcct tcctggatgt 2640
cccccgcttg tctcattggc atctgtggtg gagagttctt tgtggatggc ttgcacatga 2700
cattttctgt ttctgtaaca ctttctgtgt ggacaattct gtccttttcc tctgtggctc 2760
ccatatgggt tggataggtg acagtctagg tctccctccc aaggtgttaa tttccagcaa 2820
gggaaaactc caagatccac cccctaaact gggccagaca agtcaacacc ccttccctga 2880
ggaagccatc accactggga ccacctggcc ttcctccatt gcagcctgtg gatccctggc 2940
ctcccgcctg agttgggggt gggggtggag gcataagtct gaaaaaaaag tgggtaatag 3000
tcagcagttc cctaaagtag aattactacc caccctctgc catggacccc agcataaagc 3060
tttttgtggg gcgtgcatga cctgactctg tactggcgtt aggcttcagg agatctgtgg 3120
aatagagttt gacttcatgg atcctaaccc tctgagacct gctgctgact cccagggagc 3180
aggtcctccc tcacagtctg tgcctcagct tccccagaaa aggtagatgg aggtgagtta 3240
aatgcttgga gatcctacac ctgggctttt gcttcctttt tccttgcacg gtcagtgccc 3300
aacacagtgc ctgacattca cactgaggcc cagcatacct atctttaagc agtttggaag 3360
tgatcttctg ggggtgatgg gcgtcttaaa acggtttcca tagcaagcac cttactttta 3420
ctcacatggg atgtttgttt ggaggggggc attaaaagga ctgctggcaa ggaggagaga 3480
gactaaacgc ctccccaagg gagccatttc ttggcatagt agttaaggga gaccacttct 3540
cagcattcgg aaggaactgg ttgggagcag gtgtccacga tgtctgggga ggggtcactt 3600
cgtgtggtct tggatatcag gagtggcaga gaggcaacac attccagatg agctctgccc 3660
tttgacacct ccccccaccc caccccggct gggtcaaggc cccaaggcca catatagaag 3720
gcgtggaagg tgggcaccag ctgctgctgc caggagatcc atgctccata agccctcgcg 3780
ccagcctcgg agacagccag gctgcgcaga gcagaggaga ggaagcagcg aggtgggaga 3840
cagtttcgct tctcccaccg aggccgcgcc tgtggctgca ggcgcccgga tcgccacagt 3900
taactccctg ttatctgggc cccaatcgat ctcaggctgt aacgtttaac cctaggctga 3960
cttccccaac cgccctcccc ctctccctcc cctgcttggg tctggggact gcgttttctt 4020
cgcctcgcag aggaaggtcg gccttgcaca aatccgcccc cacgaatccc aaagaattgg 4080
agaaaacttc cactttggga ttatatgcag ttgggattat atgagcccgg ggcttgcacc 4140
atgggtacgt gaggtgaaga catctgcagc agcaggatta attagcctaa gatgggcata 4200
tggaggccac tgatggagtg aaagcgggat atgcgggccc aactctgtcc ccactgggcg 4260
cctactctgg caaggctgag ttttccttct gtctttcaat cagtacctat tgagcgctgc 4320
 
tgttccaagc attgtttcgg gttttgagtg cgtaggccat ggtctcaggg caccaaatgt 4380
acgtagtcgt tttagccccg ggactcaaga gttgaggctg atgcctgcct gagagataaa 4440
atatcctttc tcggatcagt ttcctcacct gagaaatggg aacgggaatc tccgcccctt 4500
ttctcccggg gccctagtgc ccactgaatc cattaaggag ctcttggaag ggtggggtct 4560
tggaacacgc gtctacctcc caggaccctc gactaggaat ctctggcccg ccgcgcacct 4620
gagctggggg gcgcggccaa attctccctc ccggtcctcg aagcttctgg ccccgctcta 4680
gagggggcag aaagatagca gatttccccg gaggtgcgtc tctaaaccca gaccgcctca 4740
ccaaagcggg gaggtcgtcc tttttttttt ttttttgtaa agttgcggga aataggcgaa 4800
gacggggcgc gcaagggagc tgagcctggg tggaccgcgt cagggcgcgg ggagctcgag 4860
gcgcagcggc tgcagctccg gcctcagagc ccgcggcgtc caagtggccc gagctgcgct 4920
gggagggagg cggcgggagg agggagggga gccgaggtgg agggggttgg ggggaggagg 4980
gagcggcgag tccggtccgg 5000
<212> Type : DNA
<211> Length : 5000
   SequenceName : Promotor
   SequenceDescription :

## Claims

1. An in vitro method for diagnosis of myelodysplasia del 5q (MDS), Acute Myeloid Leukemia (AML), breast cancer and Melanoma comprising the step of detection of the expression level of a nucleic acid comprising the sequence of SEQ ID NO 1 or SEQ ID NO 2 or the expression level for a protein comprising the sequence of SEQ ID NO 3.

2. The method according to claim 1 wherein the expression level of said protein is determined by the use of an antibody.

## Patentansprüche

1. Ein in vitro Verfahren zur Diagnose von Myelodysplasie del 5q (MDS), akute myeloische Leukämie (AML), Brustkrebs und Melanom umfassend den Schritt des Bestimmen des Expressionsniveaus einer Nucleinsäure umfassend die Sequenz gemäß SEQ ID NO 1 oder SEQ ID NO 2 oder des Expressionsniveaus für ein Protein umfassend die Sequenz gemäß SEQ ID NO 3.

2. Verfahren nach Anspruch 1, wobei das Expressionsniveau des Proteins bestimmt wird durch Verwendung eines Antikörpers.

## Revendications

1. Procédé in vitro pour diagnostiquer la myélodysplasie del 5q (MDS), la leucémie myéloïde aiguë (AML), le cancer du sein et le mélanome, comprenant l'étape consistant à détecter le niveau d'expression d'un acide nucléique comprenant la séquence SEQ ID NO 1 ou SEQ ID NO 2 ou le niveau d'expression d'une protéine comprenant la séquence SEQ ID NO 3.

2. Procédé selon la revendication 1, dans lequel le niveau d'expression de ladite protéine est déterminé par l'utilisation d'un anticorps.
